(19) Europäisches Patentamt
European Patent Office
Office européen des brevets

(11) EP 3 934 611 B1

(12) # EUROPÄISCHE PATENTSCHRIFT

(45) Veröffentlichungstag und Bekanntmachung des Hinweises auf die Patenterteilung:
**01.03.2023 Patentblatt 2023/09**

(21) Anmeldenummer: **20702753.3**

(22) Anmeldetag: **24.01.2020**

(51) Internationale Patentklassifikation (IPC):
***A61K 8/58*** *(2006.01)* ***A61Q 5/06*** *(2006.01)*

(52) Gemeinsame Patentklassifikation (CPC):
**A61Q 5/065; A61K 8/585**

(86) Internationale Anmeldenummer:
**PCT/EP2020/051822**

(87) Internationale Veröffentlichungsnummer:
**WO 2020/177945 (10.09.2020 Gazette 2020/37)**

(54) **MITTEL ZUR BEHANDLUNG VON KERATINISCHEN FASERN ENTHALTEND DAS REAKTIONSPRODUKT AUS ZWEI ORGANISCHEN C1-C6-ALKOXY-SILANEN UND WASSER**

PRODUCT FOR TREATING KERATIN FIBERS, CONTAINING THE REACTION PRODUCT FROM TWO ORGANIC C1-C6 ALKOXY-SILANES AND WATER

MOYENS POUR LE TRAITEMENT DE FIBRES KÉRATINIQUES CONTENANT LE PRODUIT DE RÉACTION COMPOSÉ DE DEUX ALCOXY-SILANES EN C1-C6 ORGANIQUES ET DE L'EAU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.03.2019 DE 102019203083**

(43) Veröffentlichungstag der Anmeldung:
**12.01.2022 Patentblatt 2022/02**

(73) Patentinhaber: **Henkel AG & Co. KGaA**
**40589 Düsseldorf (DE)**

(72) Erfinder:
- **LECHNER, Torsten**
  **40764 Langenfeld (DE)**
- **SCHOEPGENS, Juergen**
  **41366 Schwalmtal (DE)**

(56) Entgegenhaltungen:
**WO-A2-2012/038880**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 934 611 B1

## Beschreibung

Die vorliegende Anmeldung liegt auf dem Gebiet der Kosmetik und betrifft eine kosmetische Zusammensetzung zur Behandlung von keratinischem Material, enthaltend ein Produkt (a), das durch Mischen eines bestimmten $C_1$-$C_6$-Alkoxy-Silans (a1) der Formel (I) und eines bestimmten $C_1$-$C_6$-Alkoxy-Silans (a2) der Formel (II) mit Wasser (a3) erhalten wird. Weiterhin ist die Zusammensetzung dadurch gekennzeichnet, dass ihr Gehalt an $C_1$-$C_6$-Alkoholen (b) und Wasser (c) bei jeweils höchstens 10,0 Gew.-% liegt.

Ein zweiter Gegenstand der vorliegenden Erfindung ist eine Mehrkomponenten-Verpackungseinheit (Kit-of-parts) zum Färben von keratinischem Material, welche getrennt konfektioniert in zwei Verpackungseinheiten die kosmetischen Zubereitungen (A) und (B) umfasst, wobei es sich bei der Zubereitung (A) um eine Zubereitung des ersten Erfindungsgegenstandes handelt und die Zubereitung (B) mindestens eine farbgebende Verbindung enthält.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung einer Kombination aus Pigment, organischer Silicium-Verbindung, hydrophobem Polymer und einem Lösungsmittel auf Haaren Färbungen erzeugen lassen, die gegenüber Schamponierungen besonders widerstandsfähig sind.

Bei den in der EP 2168633 B1 eingesetzten organischen Silicium-Verbindungen handelt es sich um reaktive Verbindungen aus der Klasse der Alkoxy-Silane. Diese Alkoxy-Silane hydrolysieren in Gegenwart von Wasser mit hoher Geschwindigkeit und bilden - abhängig von den jeweils eingesetzten Mengen an Alkoxy-Silan und Wasser - Hydrolyseprodukte und/oder Kondensationsprodukte aus. Der Einfluss der in dieser Reaktion eingesetzten Wassermenge auf die Eigenschaften des Hydrolyse- bzw. Kondensationsproduktes werden beispielsweise in WO 2013068979 A2 beschrieben.

Auch bekannt ist aus WO 2012/038880 A2 eine Zusammensetzung zur kosmetischen Behandlung von Keratinfasern und Zähnen, das durch Mischen (a) eines Alkylalkoxysilans, (b) eines Alkylsilans, (c) einer Säure und (d) Wasser erhalten wird. Die Zusammensetzungen, die in den Beispielen offenbart werden, enthalten ca. 39% Ethanol und ca. 30% Wasser.

Wenn diese Hydrolyse- bzw. Kondensationsprodukte auf keratinischem Material angewendet werden, bildet sich auf dem Keratinmaterial ein Film oder auch ein Coating aus, welches das Keratinmaterial vollständig umhüllt und auf diese Weise die Eigenschaften des Keratinmaterials stark beeinflusst. Mögliche Anwendungsbereiche sind beispielsweise das permanente Styling oder auch die permanente Formveränderung von Keratinfasern. Hierbei werden die Keratinfasern mechanisch in die gewünschte Form gebracht und in dieser Form dann durch Ausbildung des vorbeschriebenen Coatings fixiert. Eine weitere ganz besonders gut geeignete Anwendungsmöglichkeit ist die Färbung von Keratinmaterial; im Rahmen dieser Anwendung wird das Coating bzw. der Film in Gegenwart einer farbgebenden Verbindung, zum Beispiel eines Pigments, erzeugt. Der durch das Pigment gefärbte Film verbleibt auf dem Keratinmaterial bzw. den Keratinfasern und resultiert in überraschend waschbeständigen Färbungen.

Der große Vorteil des auf Alkoxy-Silanen basierenden Färbeprinzips liegt darin, dass die hohe Reaktivität dieser Verbindungsklasse ein sehr schnelles Coating ermöglicht. So können bereits nach sehr kurzen Anwendungszeiträumen

von nur wenigen Minuten extrem gute Färbeergebnisse erzielt werden. Neben diesen Vorteilen birgt die hohe Reaktivität der Alkoxy-Silane jedoch auch einige Nachteile. So können bereits geringfügige Änderungen der Produktions- und Anwendungsbedingungen, wie beispielsweise die Änderung von Luftfeuchtigkeit und/oder Temperatur, zu starken Schwankungen der Produktleistung führen. Vor allem haben die zu dieser Erfindung führenden Arbeiten gezeigt, dass die Alkoxy-Silane extrem sensibel auf die Bedingungen reagieren, die bei der Herstellung der Keratinbehandlungsmittel herrschen. Weichen diese Herstellungsbedingungen nur geringfügig von ihrem optimalen Wertebereich ab, so kann dies zum teilweisen oder sogar vollständigen Verlust der Produktleistung führen. Vor allem die Färbeleistung eines unter nicht optimalen Bedingungen hergestellten Färbemittels auf Alkoxy-Silan-Basis kann dramatisch sinken. Hierbei hat sich herausgestellt, dass insbesondere der Gehalt an $C_1$-$C_6$-Alkoholen und an Wasser im Keratinbehandlungs- bzw. Färbemittel einen großen Einfluss dessen Produktleistung nimmt.

Es war die Aufgabe der vorliegenden Anmeldung, eine Zubereitung zur Behandlung von Keratinmaterial aufzufinden, welche die reaktiven Alkoxy-Silane in optimaler Abmischung und Zusammensetzung enthält. Hierbei sollten die zur Herstellung des Mittels eingesetzten Alkoxy-Silane auf gezielte Weise so hydrolysiert und kondensiert werden, dass Zusammensetzungen mit den optimalen anwendungstechnischen Eigenschaften erhalten werden konnten. Insbesondere sollten die auf diesem Wege hergestellten Mittel eine verbesserte Färbeleistung besitzen, d.h. bei ihrer Anwendung in einem Färbeverfahren sollten Färbungen mit höherer Farbintensität und verbesserten Echtheitseigenschaften, insbesondere mit einer verbesserten Waschechtheit und einer verbesserten Reibechtheit, erzielt werden.

Überraschenderweise hat sich nun herausgestellt, dass die vorgenannte Aufgabe hervorragend gelöst werden kann, wenn die Zusammensetzung zur Behandlung des Keratinmaterials durch Abmischung von zwei Silanen bestimmter Strukturformeln (I) und (II) mit Wasser hergestellt wird, wobei alle drei Reaktionspartner (d.h. die Silane der Formeln (I) und(II) sowie das Wasser) bevorzugt in bestimmten Molverhältnissen miteinander zur Reaktion gebracht werden. Wesentlich für die Zusammensetzung ist weiterhin, dass die bei der Reaktion der Alkoxy-Silane freigesetzten Alkohole so vollständig wie möglich aus dem Reaktionsgemisch entfernt werden. Weiterhin als wesentlich hat sich herausgestellt, dass der Wassergehalt in der Zubereitung eine bestimmte Maximalmenge nicht überschreiten darf.

Ein erster Gegenstand der vorliegenden Erfindung ist eine kosmetische Zusammensetzung zur Behandlung von keratinischem Material, insbesondere keratinischen Fasern, enthaltend

(a) ein Produkt, das erhalten wird durch Mischen von

(a1) einem oder mehreren organischen $C_1$-$C_6$-Alkoxy-Silanen der Formel (I)

$$H_2N\text{-}L\text{-}Si(OR_1)_3 \qquad (I)$$

wobei

- L für eine lineare oder verzweigte, zweiwertige $C_1$-$C_{20}$-Alkylengruppe steht,
- $R_1$ für eine $C_1$-$C_6$-Alkylgruppe steht,
  mit

(a2) einem oder mehreren organischen $C_1$-$C_6$-Alkoxy-Silanen der Formel (II)

$$R_2\text{-}Si(OR_3)_3 \qquad (II)$$

wobei

- $R_2$ für eine $C_1$-$C_{12}$-Alkylgruppe steht, und
- R3 für eine $C_1$-$C_6$-Alkylgruppe steht, und

(a3) Wasser, und - bezogen auf das Gesamtgewicht der Zusammensetzung -

(b) einen oder mehrere $C_1$-$C_6$-Alkohole in einer Gesamtmenge von 0,001 bis 10,0 Gew.-% und
(c) 0,001 bis 10,0 Gew.-% Wasser.

Es hat sich gezeigt, dass Haarbehandlungsmittel mit der oben genannten Zusammensetzung bei Einsatz in einem Färbeprozess zu sehr intensiven und gleichmäßigen Färbungen mit sehr guter Reibechtheit und Waschechtheit führten.

Mittel zur Behandlung von keratinischem Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Unter Mitteln zur Behandlung von keratinischem Material werden beispielsweise Mittel zur Färbung des Keratinmaterials, Mittel zur Umformung oder Formgebung von keratinischem Mateiral, insbesondere keratinischen Fasern, oder auch Mittel zur Konditionierung bzw. zur Pflege des keratinsichen Materials verstanden. Besonders gute Eignung zeige die über das erfindungsgemäße Verfahren hergestellten Mittel zur Färbung von keratinischem Material, inbesondere zur Färbung von keratinischen Fasern, bei denen es sich besonders bevorzugt um menschliche Haare handelt.

Der Begriff "Mittel zur Färbung" wird im Rahmen dieser Erfindung für eine durch Einsatz von farbgebenden Verbindungen, wie beispielsweise thermochromen und photochromen Farbstoffen, Pigmenten, Mica, direktziehenden Farbstoffen und/oder Oxidationsfarbstoffen hervorgerufene Farbgebung des Keratinmaterials, insbesondere des Haares, verwendet. Bei dieser Färbung lagern sich die vorgenannten farbgebenden Verbindungen in einem besonders homogenen und glatten Film an der Oberfläche des Keratinmaterials ab oder diffundieren in die Keratinfaser hinein. Der Film bildet sich *in situ* durch Oligomerisierung bzw. Polymerisierung des oder der organischen Siliciumverbindungen, und durch die Wechselwirkung von farbgebender Verbindung und organischer Siliciumverbindung und optional weiteren Bestandteilen, wie beispielsweise einem filmbildenden, hydrophilen Polymer.

Produkt aus $C_1$-$C_6$-Alkoxv-Silanen (a1) mit $C_1$-$C_6$-Alkoxv-Silanen (a2) und Wasser (a3)

Als ersten erfindungswesentlichen Bestandteil (a) enthält die erfindungsgemäße Zusammensetzung ein Produkt, das durch Vermischen von einem $C_1$-$C_6$-Alkoxy-Silan (a1) der Formel (I) mit einem $C_1$-$C_6$-Alkoxy-Silan (a2) der Formel (II) und Wasser (a3) erhalten wird.

Bei den $C_1$-$C_6$-Alkoxy-Silanen der Formeln (I) und (II) handelt es sich jeweils um sehr reaktive Verbindungen, die in Gegenwart von Wasser eine Hydrolyse-Reaktion eingehen. Diese Hydrolyse-Reaktion verläuft exotherm und startet bei Kontakt der Silane (I) bzw. (II) mit Wasser.

Bei dem oder den organischen $C_1$-$C_6$-Alkoxy-Silanen handelt es sich um organische, nicht polymere Siliciumverbindungen, die bevorzugt aus der Gruppe der Silane mit einem, zwei oder drei Siliciumatomen ausgewählt ist.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die erfindungsgemäßen organische Siliciumverbindungen sind bevorzugt Verbindungen, die ein bis drei Silicumatome enthalten. Besonders bevorzugt enthalten die organische Siliciumverbindungen ein oder zwei Siliciumatome.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die Wasserstoff-Atome ganz oder teilweise durch organische Gruppen wie beispielsweise (substituierte) Alkylgruppen und/oder Alkoxygruppen ersetzt.

Kennzeichnend für die erfindungsgemäßen $C_1$-$C_6$-Alkoxy-Silane ist, dass drei $C_1$-$C_6$-Alkoxygruppen direkt an das Siliciumatom gebunden vorliegen. Die erfindungsgemäßen $C_1$-$C_6$-Alkoxy-Silane umfassen damit mindestens eine Struktureinheit R'Si-(O-$C_1$-$C_6$-Alkyl)$_3$ wobei der Rest R' jeweils für den entsprechenden strukturellen Einheiten bei den $C_1$-$C_6$-Alkoxy-Silane der Formeln (I) und (II) stehen.

Diese an das Siliciumatom gebundenen $C_1$-$C_6$-Alkoxygruppen sind sehr reaktiv und werden in Anwesenheit von Wasser mit hoher Geschwindigkeit hydrolysiert, wobei die Reaktionsgeschwindigkeit unter anderem auch von der Anzahl der hydrolysierbaren Gruppen pro Molekül abhängt. Handelt es sich bei der hydrolysierbaren $C_1$-$C_6$-Alkoxy-Gruppe um eine Ethoxygruppe, so enthält die organische Siliciumverbindung bevorzugt eine Struktureinheit R'Si(O-$CH_2$-$CH_3$)$_3$. Der Reste R' steht hierbei wieder exemplarisch für die vierte an das Siliciumatom gebundene Struktureinheit.

Bei den $C_1$-$C_6$-Alkoxy-Silanen (a1) der Formel (I)

$$H_2N\text{-}L\text{-}Si(OR_1)_3 \qquad (I)$$

steht

- L für eine lineare oder verzweigte, zweiwertige $C_1$-$C_{20}$-Alkylengruppe, und
- $R_1$ für eine $C_1$-$C_6$-Alkylgruppe.

Die Substituenten $R_1$, $R_2$, $R_3$ und L in den Verbindungen der Formel (I) und (II) sind nachstehend beispielhaft

erläutert:
Beispiele für eine $C_1$-$C_6$-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine lineare zweiwertige $C_1$-$C_{20}$-Alkylengruppe sind beispielsweise die Methylen-gruppe ($-CH_2-$), die Ethylengruppe ($-CH_2-CH_2-$), die Propylengruppe ($-CH_2-CH_2-CH_2-$) und die Butylengruppe ($-CH_2-CH_2-CH_2-CH_2-$). Die Propylengruppe ($-CH_2-CH_2-CH_2-$) ist besonders bevorzugt. Ab einer Kettenlänge von 3 C-Atomen können zweiwertige Alkylengruppen auch verzweigt sein. Beispiele für verzweigte, zweiwertige $C_3$-$C_{20}$-Alkylengruppen sind ($-CH_2-CH(CH_3)-$) und ($-CH_2-CH(CH_3)-CH_2-$).

Im Mittelteil der organischen Siliciumverbindung der Formel (I) befindet sich die Struktureinheit oder der Linker -L- der für eine lineare oder verzweigte, zweiwertige $C_1$-$C_{20}$-Alkylengruppe steht. Die zweiwertige $C_1$-$C_{20}$-Alkylengruppe kann alternativ auch als eine divalente oder zweibindige $C_1$-$C_{20}$-Alkylengruppe bezeichnet werden, womit gemeint ist, dass jede Gruppierung -L- zwei Bindungen eingehen kann.

Bevorzugt steht -L- für eine lineare, zweiwertige $C_1$-$C_{20}$-Alkylengruppe. Weiter bevorzugt steht -L- für eine lineare zweiwertige $C_1$-$C_6$-Alkylengruppe. Besonders bevorzugt steht -L- für eine Methylengruppe ($-CH_2-$), eine Ethylengruppe ($-CH_2-CH_2-$), eine Propylengruppe ($-CH_2-CH_2-CH_2-$) oder eine Butylengruppe ($-CH_2-CH_2-CH_2-CH_2-$). Ganz besonders bevorzugt steht L für eine Propylengruppe ($-CH_2-CH_2-CH_2-$).

Die erfindungsgemäßen organischen Siliciumverbindungen der Formel (I)

$$H_2N\text{-}L\text{-}Si(OR_1)_3 \qquad (I)$$

tragen jeweils ein einem Ende die Silicium-haltige Gruppierung $-Si(OR_1)_3$.

Hierbei steht der Rest $R_1$ für eine $C_1$-$C_6$-Alkylgruppe. Besonders bevorzugt steht $R_1$ für eine Methylgruppe oder eine Ethylgruppe.

Haarbehandlungsmittel mit besonders guten Eigenschaften konnten hergestellt werden, wenn zum Erhalt des Produktes (a) mindestens ein organisches $C_1$-$C_6$-Alkoxy-Silan (a1) der Formel (I) mit einem $C_1$-$C_6$-Alkoxy-Silan (a2) der Formel (II) und Wasser zur Reaktion gebracht wurde, bei dem R1 für eine Methylgruppe oder für eine Ethylgruppe steht.

Zur Lösung der erfindungsgemäßen Aufgabenstellung besonders gut geeignete organische $C_1$-$C_6$-Alkoxy-Silane (a1) der Formel (I) sind

- (3-Aminopropyl)triethoxysilan

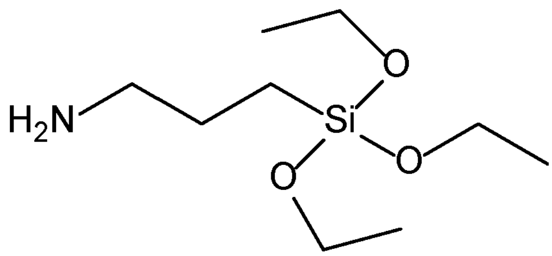

- (3-Aminopropyl)trimethoxysilan

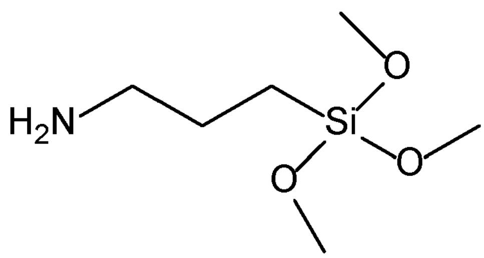

- (2-Aminoethyl)triethoxysilan

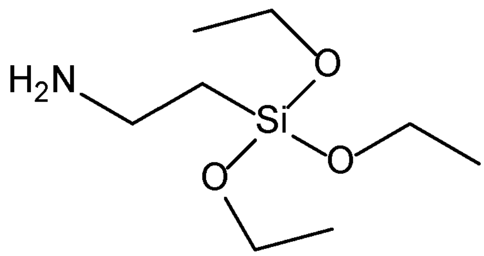

- (2-Aminoethyl)trimethoxysilan

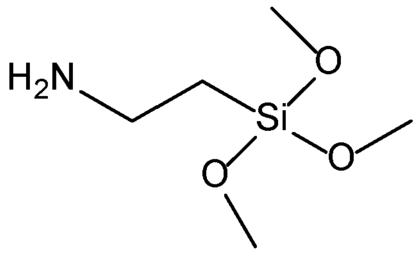

- (3-Dimethylaminopropyl)triethoxysilan

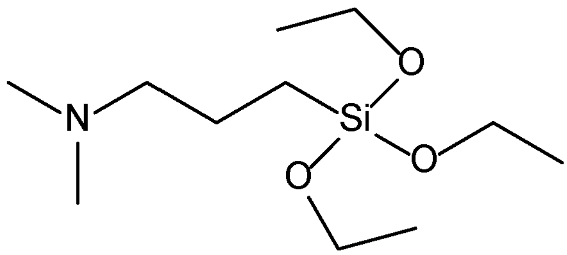

- (3-Dimethylaminopropyl)trimethoxysilan

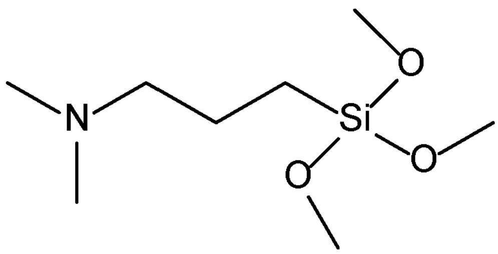

- (2-Dimethylaminoethyl)triethoxysilan.

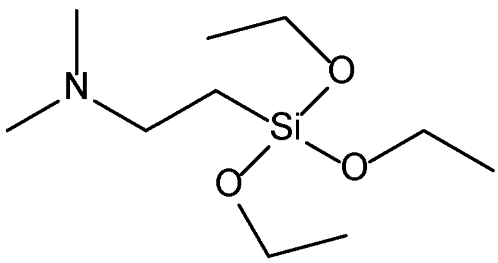

- (2-Dimethylaminoethyl)trimethoxysilan und/oder

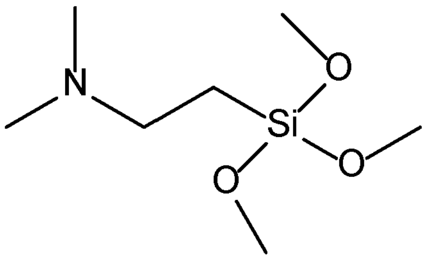

In einer weiteren bevorzugten Ausführungsform ist ein erfindungsgemäße Zusammensetzung dadurch gekennzeichnet, dass sie ein Produkt (a) enthält, das durch Abmischung von (a1) einem oder mehreren organischen $C_1$-$C_6$-Alkoxy-Silanen der Formel (I) aus der Gruppe aus

- (3-Aminopropyl)triethoxysilan
- (3-Aminopropyl)trimethoxysilan
- (2-Aminoethyl)triethoxysilan
- (2-Aminoethyl)trimethoxysilan
- (3-Dimethylaminopropyl)triethoxysilan
- (3-Dimethylaminopropyl)trimethoxysilan
- (2-Dimethylaminoethyl)triethoxysilan.
- (2-Dimethylaminoethyl)trimethoxysilan und/oder

erhalten wurde.

Die vorgenannten organische Siliciumverbindungen der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Bei den $C_1$-$C_6$-Alkoxy-Silanen (a2) der Formel (II)

$$R_2\text{-Si}(OR_3)_3 \qquad (II)$$

steht

- $R_2$ für eine $C_1$-$C_{12}$-Alkylgruppe, und
- $R_3$ für eine $C_1$-$C_6$-Alkylgruppe.

In den organischen $C_1$-$C_6$-Alkoxy-Silanen der Formel (II) steht der Rest $R_2$ für eine $C_1$-$C_{12}$-Alkylgruppe. Diese $C_1$-$C_{12}$-Alkylgruppe ist gesättigt und kann linear oder verzweigt sein. Bevorzugt steht $R_2$ für eine lineare $C_1$-$C_8$-Alkylgruppe. Bevorzugt steht $R_2$ für eine Methylgruppe, eine Ethylgruppe, eine n-Propylgruppe, eine n-Butylgruppe, eine n-Pentylgruppe, eine n-Hexylgruppe, eine n-Octylgruppe oder eine n-Dodecylgruppe. Besonders bevorzugt steht $R_9$ für eine Methylgruppe, eine Ethylgrurppe, eine n-Hexylgruppe oder eine n-Octylgruppe.

In den organischen Siliciumverbindungen der Formel (II) steht der Rest $R_3$ für eine $C_1$-$C_6$-Alkylgruppe. Besonders bevorzugt steht $R_3$ für eine Methylgruppe oder für eine Ethylgruppe.

Zur Lösung der erfindungsgemäßen Aufgabenstellung besonders gut geeignete organische Silicumverbindungen der Formel (II) sind

- Methyltrimethoxysilan

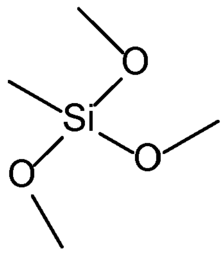

- Methyltriethoxysilan

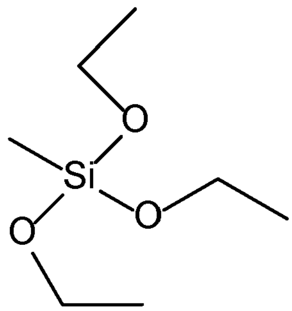

- Ethyltrimethoxysilan

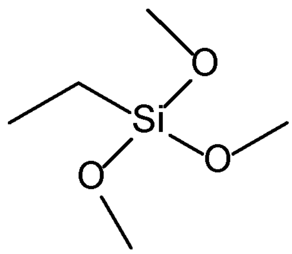

- Ethyltriethoxysilan

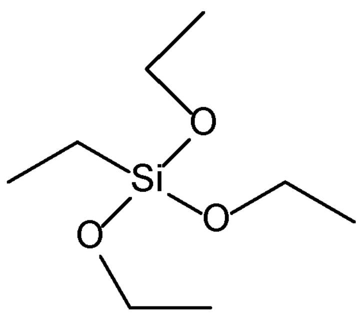

- n-Propyltrimethoxysilan (auch bezeichnet als Propyltrimethoxysilan)

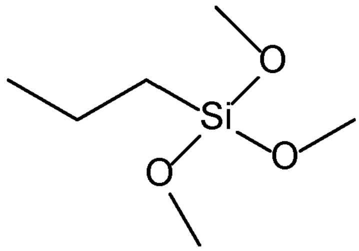

- n-Propyltriethoxysilan (auch bezeichnet als Propyltriethoxysilan)

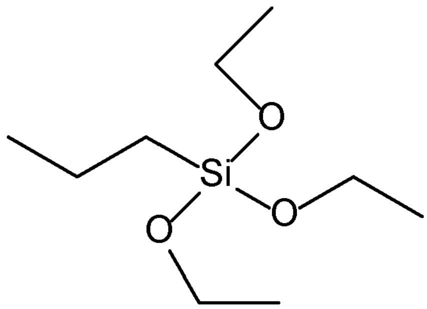

- n-Hexyltrimethoxysilan (auch bezeichnet als Hexyltrimethoxysilan)

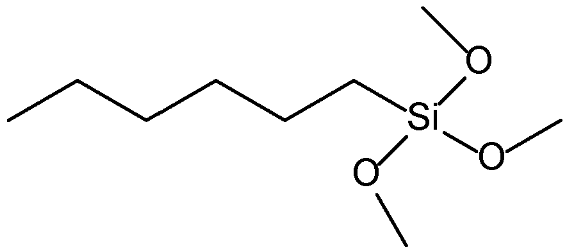

- n-Hexyltriethoxysilan (auch bezeichnet als Hexyltriethoxysilan)

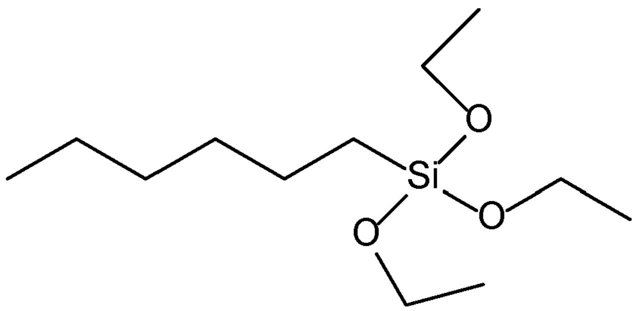

- n-Octyltrimethoxysilan (auch bezeichnet als Octyltrimethoxysilan)

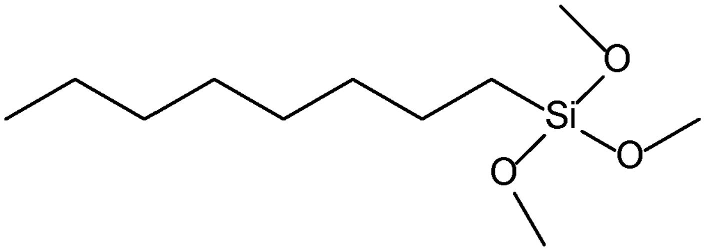

- n-Octyltriethoxysilan (auch bezeichnet als Octyltriethoxysilan)

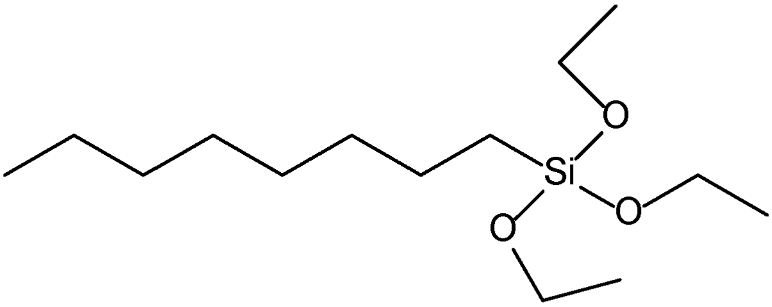

- n-Dodecyltrimethoxysilan (auch bezeichnet als Dodecyltrimethoxysilan) und/oder

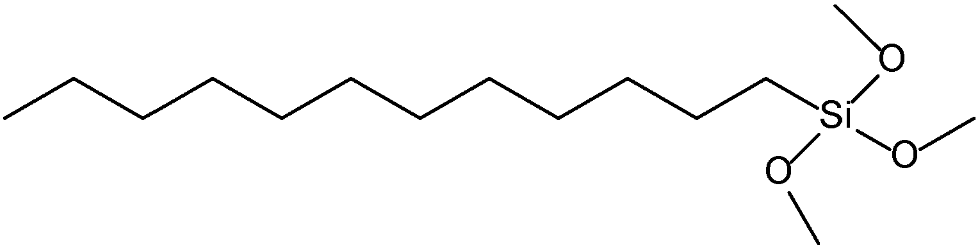

- n-Dodecyltriethoxysilan (auch bezeichnet als Dodecyltriethoxysilan)

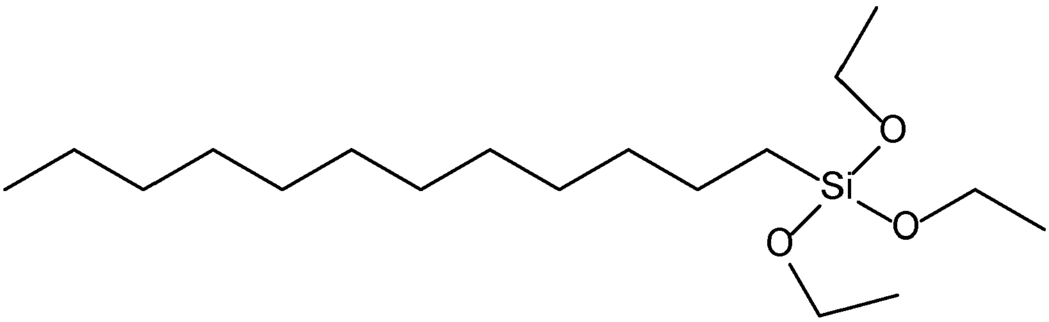

In einer weiteren bevorzugten Ausführungsform ist ein erfindungsgemäße Zusammensetzung dadurch gekennzeichnet, dass sie ein Produkt (a) enthält, das durch Abmischung von (a2) einem oder mehreren organischen $C_1$-$C_6$-Alkoxy-Silanen der Formel (II) aus der Gruppe aus

- Methyltrimethoxysilan
- Methyltriethoxysilan
- Ethyltrimethoxysilan
- Ethyltriethoxysilan
- Propyltrimethoxysilan

- Propyltriethoxysilan
- Hexyltrimethoxysilan
- Hexyltriethoxysilan
- Octyltrimethoxysilan
- Octyltriethoxysilan
- Dodecyltrimethoxysilan und/oder
- Dodecyltriethoxysilan,

mit Wasser zur Reaktion gebracht werden.

Bei Abmischung ganz bestimmter $C_1$-$C_6$-Alkoxy-Silanen der Formel (I) und (II) mit Wasser wurde ein Produkt (a) mit ganz besonders vorteilhaften Eigenschaften erhalten.

Im Rahmen einer Ausführungsform ganz besonders bevorzugt ist eine kosmetische Zusammensetzung zur Behandlung von keratinischem Material, insbesondere keratinischen Fasern, enthaltend

(a) ein Produkt, das erhalten wird durch Mischen von

(a1) (3-Aminopropyl)triethoxysilan mit
(a2) Methyltrimethoxysilan und
(a3) Wasser.

Im Rahmen einer Ausführungsform ganz besonders bevorzugt ist eine kosmetische Zusammensetzung zur Behandlung von keratinischem Material, insbesondere keratinischen Fasern, enthaltend

(a) ein Produkt, das erhalten wird durch Mischen von

(a1) (3-Aminopropyl)triethoxysilan mit
(a2) Ethyltriethoxysilan und
(a3) Wasser.

Im Rahmen einer Ausführungsform ganz besonders bevorzugt ist eine kosmetische Zusammensetzung zur Behandlung von keratinischem Material, insbesondere keratinischen Fasern, enthaltend

(a) ein Produkt, das erhalten wird durch Mischen von

(a1) (3-Aminopropyl)triethoxysilan mit
(a2) Methyltriethoxysilan und
(a3) Wasser.

Im Rahmen einer Ausführungsform ganz besonders bevorzugt ist eine kosmetische Zusammensetzung zur Behandlung von keratinischem Material, insbesondere keratinischen Fasern, enthaltend

(a) ein Produkt, das erhalten wird durch Mischen von

(a1) (3-Aminopropyl)triethoxysilan mit
(a2) Propyltriethoxysilan und
(a3) Wasser.

Im Rahmen einer Ausführungsform ganz besonders bevorzugt ist eine kosmetische Zusammensetzung zur Behandlung von keratinischem Material, insbesondere keratinischen Fasern, enthaltend

(a) ein Produkt, das erhalten wird durch Mischen von

(a1) (3-Aminopropyl)triethoxysilan mit
(a2) Hexyltriethoxysilan und
(a3) Wasser.

Im Rahmen einer Ausführungsform ganz besonders bevorzugt ist eine kosmetische Zusammensetzung zur Behandlung von keratinischem Material, insbesondere keratinischen Fasern, enthaltend

(a) ein Produkt, das erhalten wird durch Mischen von

(a1) (3-Aminopropyl)triethoxysilan mit
(a2) Octyltriethoxysilan und
(a3) Wasser.

Da beide Silane der Strukturgruppen (a1) und (a2) bei der Hydrolyse jeweils mit Wasser und bei einer späteren Kondensation auch miteinander reagieren können, sind die zum Produkt (a) führenden Reaktionen sehr komplex, und mit der Abmischung entstehen Gemische von monomeren und oligomeren Silan-Kondensaten. Es wird vermutet, dass bei Abmischung von (a1) mit (a2) und (a3) insbesondere die folgenden Reaktionen initiiert werden:

Hydrolyse von $C_1$-$C_6$-Alkoxy-Silan der Formel (I) mit Wasser (Reaktionsschema am Beispiel von 3-Aminopropyltriethoxysilan):

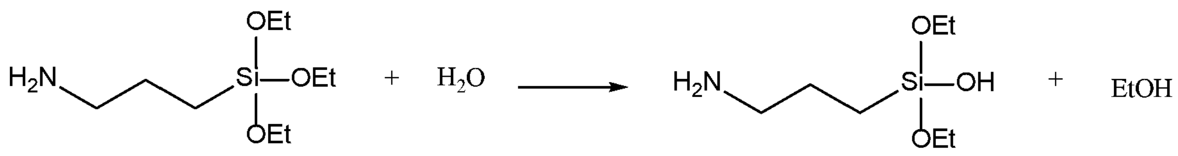

In Abhängigkeit von der eingesetzten Menge an Wasser (a3) kann die Hydrolyse-Reaktion auch mehrfach pro eingesetztem $C_1$-$C_6$-Alkoxy-Silan (a1) stattfinden:

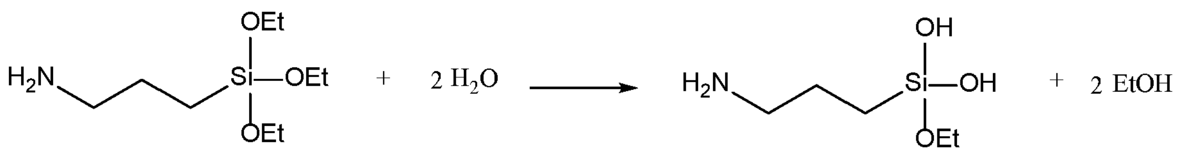

bzw.

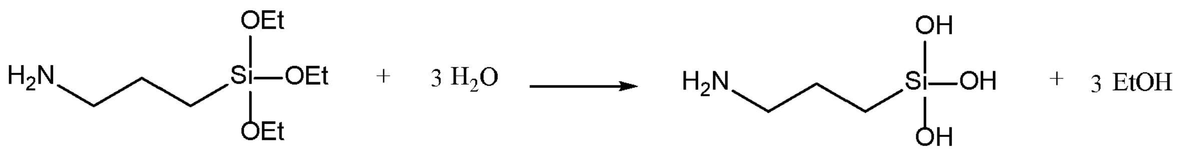

Hydrolyse von $C_1$-$C_6$-Alkoxy-Silan der Formel (II) mit Wasser (Reaktionsschema am Beispiel von Methyltrimethoxysilan):

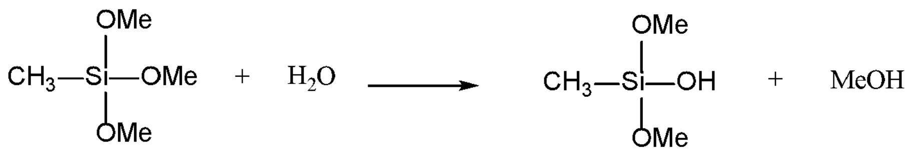

In Abhängigkeit von der eingesetzten Menge an Wasser (a3) kann die Hydrolyse-Reaktion auch mehrfach pro eingesetztem $C_1$-$C_6$-Alkoxy-Silan (a2) stattfinden:

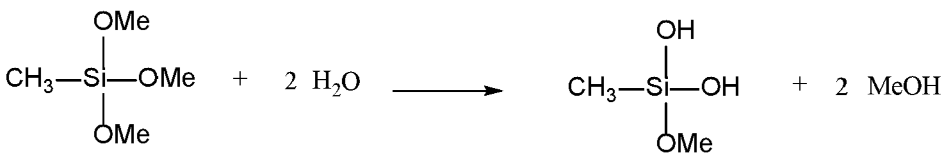

bzw.

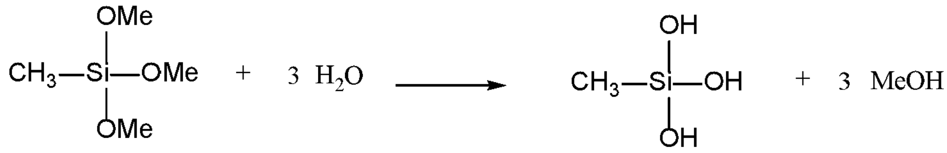

Da die Hydrolyse-Reaktion exotherm ist, hat es sich als besonders vorteilhaft herausgestellt, das Reaktionsgemisch aus den $C_1$-$C_6$-Alkoxy-Silanen (a1) und (a2) und Wasser (a3) zur verbesserten Wärmeabfuhr zu rühren bzw. zu durchmischen.

Die zum Produkt (a) führende Reaktion der organischen $C_1$-$C_6$-Alkoxy-Silane (a1) und (a2) mit Wasser (a3) kann auf verschiedenen Wegen stattfinden. Eine Möglichkeit ist es, die gewünschte Wassermenge (a3) im Reaktionsgefäß oder Reaktor vorzulegen und im Anschluss daran das oder die $C_1$-$C_6$-Alkoxy-Silane (a1) und (a2) hinzuzufügen.

Im Rahmen einer weiteren Ausführungsform werden in einem Reaktionsgefäß oder Reaktor zunächst die entsprechenden Mengen an $C_1$-$C_6$-Alkoxy-Silanen (a1) mit den $C_1$-$C_6$-Alkoxy-Silanen (a2) vermischt, und im Anschluss daran wird die gewünschte Menge an Wasser (a3) hinzugegeben.

Das Wasser kann kontinuierlich, in Teilmengen oder direkt als Gesamtmenge zugegeben werden. Um die erforderliche Temperaturkontrolle zu gewährleisten, wird die Reaktionsmischung bevorzugt gekühlt und/oder die Zugabemenge und -geschwindigkeit des Wassers angepasst. Zur Einhaltung der gewünschten Temperaturbereiche hat es sich als ganz besonders gut geeignet herausgestellt, die notwendige Wassermenge kontinuierlich tropfenweise zu einem Gemisch aus Silanen der Formeln (I) und (II) hinzuzugeben.

Je nach Menge der eingesetzten Silane kann die Zugabe und Reaktion über einen Zeitraum von 2 Minuten bis zu 72 Stunden erfolgen.

Aufgrund der hohen Reaktivität der $C_1$-$C_6$-Alkoxy-Silane (a1) und (a2) können bei ihrer Reaktion mit Wasser (a3) komplexe Gemisch aus hydrolysierten bzw. kondensierten Silanen entstehen. Die genaue Zusammensetzung dieser Gemische wird vor allem durch die Molmengen bestimmt, in denen die Bestandteile (a1), (a2) und (a3) jeweils in der zum Produkt (a) führenden Reaktion eingesetzt werden.

Die zu dieser Erfindung führenden Arbeiten haben gezeigt, dass bei Anwendung der erfindungsgemäßen Zusammensetzung auf dem Keratinmaterial insbesondere dann ein stabiles und widerstandsfähiges Coating erzeugt werden konnte, wenn die $C_1$-$C_6$-Alkoxy-Silane (a1) und (a2) und Wasser (a3) in bestimmten Molverhältnissen miteinander vermischt und demzufolge auch in bestimmten Molverhältnissen miteinander zur Reaktion gebracht wurden.

Es hat sich als ganz besonders bevorzugt herausgestellt, die organischen $C_1$-$C_6$-Alkoxy-Silane (a2) im Vergleich zu den organischen $C_1$-$C_6$-Alkoxy-Silanen (a1) in einem molaren Überschuss einzusetzen. Ganz besonders bevorzugt werden die Silane (a2) und (a1) in einem Molverhältnis (a2)/(a1) von 3,0 bis 8,0, bevorzugt von 3,5 bis 7,5, weiter bevorzugt von 4,0 bis 7,0, noch weiter bevorzugt von 4,5 bis 6,5 und ganz besonders bevorzugt von 5,4 bis 5,9 miteinander vermischt.

Durch Einhaltung dieser Molverhältnisse (a2)/(a1) in der zum Produkt (a) führenden Reaktion konnte sichergestellt werden, dass ein Silan-Kondensat entsteht, welches die Aminoalkylgruppen (d.h. die aus den Silanen der Formel (I) stammenden Struktureinheiten -L-NH2) in optimalem Abstand zueinander enthält. In diesem Zusammenhang wird vermutet, dass die Aminoalkyl-gruppen in dem Silan-Kondensat als Linker fungieren, über welche die Adhäsion zum keratinischen Material zustande kommt. Enthält das Silan-Kondensat zu wenige von diesen Linker-Einheiten, so besteht die Gefahr, dass das Silan-Coating bei Anwendung der erfindungsgemäßen Zubereitung nicht optimal am Keratinmaterial anhaftet. Sind im Silan-Kondensat jedoch zu viele von diesen Linker-Einheiten vorhanden, scheint hierdurch eine so sperrige Molekülgeometrie zu entstehen, dass auch in diesem Fall die Adhäsion zwischen Silan-Coating und Keratinmaterial nicht optimal verläuft.

<u>Beispiel:</u>

| | | |
|---|---|---|
| (a1) | (3-Aminopropyl)triethoxysilan | molare Masse = 221,37 g/mol |
| (a2) | Methyltrimethoxysilan | molare Masse = 136,22 g/mol |

In einem Reaktionsgefäß wurden 266,6 g (3-Aminopropyl)triethoxysilan (1,204 mol) vorgelegt. Unter Rühren wurden bei Raumtemperatur 933,4 g Methyltrimethoxysilan (6,852 mol) hinzugegeben.

Das Molverhältnis (a2)/(a1) beträgt (6,852 mol) / (1,204 mol) = 5,69

Beispiel:

| | | | |
|---|---|---|---|
| (a1) | (3-Aminopropyl)triethoxysilan | molare Masse = 221,37 g/mol |
| (a2) | Methyltrimethoxysilan | molare Masse = 136,22 g/mol |
| (a2) | Ethyltriethoxysilan | molare Masse = 192.33 g/mol |

In einem Reaktionsgefäß wurden 266,6 g (3-Aminopropyl)triethoxysilan (1,204 mol) vorgelegt. Unter Rühren wurden bei Raumtemperatur 466,6 g Methyltrimethoxysilan (3,426 mol) und 658,922 g (3,426 mol) Ethyltriethoxysilan hinzugegeben.

Das Molverhältnis (a2)/(a1) beträgt (3,426 mol + 3,426 mol) / (1,204 mol) = 5,69

Im Rahmen einer weiteren ganz besonders bevorzugten Ausführungsform ist eine kosmetische Zusammensetzung zur Behandlung von keratinischem Material dadurch gekennzeichnet, dass die organischen $C_1$-$C_6$-Alkoxy-Silane (a1) und die organischen $C_1$-$C_6$-Alkoxy-Silane (a2) zum Erhalt des Produktes (a) in einem Molverhältnis (a2)/(a1) von 3,0 bis 8,0, bevorzugt von 3,5 bis 7,5, weiter bevorzugt von 4,0 bis 7,0, noch weiter bevorzugt von 4,5 bis 6,5 und ganz besonders bevorzugt von 5,4 bis 5,9 miteinander vermischt werden.

Hierbei sind die organischen $C_1$-$C_6$-Alkoxy-Silane (a1) die organischen $C_1$-$C_6$-Alkoxy-Silane der Formel (I). Die organischen $C_1$-$C_6$-Alkoxy-Silane (a2) sind die organischen $C_1$-$C_6$-Alkoxy-Silane der Formel (II).

Zur Herstellung von Mitteln, die ein besonders gutes Coating auf dem Keratinmaterial erzeugen, hat es sich als explizit ganz besonders bevorzugt herausgestellt, dass Wasser (a3) zur Herstellung des Produktes (a) in einer unterstöchiometrischen Menge einzusetzen. In diesem Fall liegt die Einsatzmenge an Wasser unterhalb der Menge, die theoretisch erforderlich wäre, um alle vorhandenen hydrolysierbaren $C_1$-$C_6$-Alkoxy-Gruppen an den Si-Atomen, d.h. die Alkoxysilangruppen, zu hydrolysieren. Ganz besonders bevorzugt ist demzufolge die partielle Hydrolyse der organischem $C_1$-$C_6$-Alkoxy-Silane.

Auch die $C_1$-$C_6$-Alkoxy-Silane (a1) und Wasser (a3) werden bevorzugt in bestimmten Molverhältnissen miteinander zur Reaktion gebracht. Die besten Ergebnisse konnten erhalten werden, wenn die organischen $C_1$-$C_6$-Alkoxy-Silane (a1) und Wasser (a3) zum Erhalt des Produktes (a) in einem Molverhältnis (a3)/(a1) von 4,0 bis 9,5, bevorzugt von 4,5 bis 9,0, weiter bevorzugt von 5,0 bis 8,5, noch weiter bevorzugt von 5,5 bis 8,0 und ganz besonders bevorzugt von 5,8 bis 6,4 miteinander vermischt wurden. Bei Einhaltung dieser Molverhältnisse wird Wasser (a3) im Vergleich zu den $C_1$-$C_6$-Alkoxy-Silanen (a1) ganz besonders in einem 5,8 bis 6,4-fachen molaren Überschuss eingesetzt.

Beispiel

| | | |
|---|---|---|
| (a1) | (3-Aminopropyl)triethoxysilan | molare Masse = 221,37 g/mol |
| (a3) | Wasser | molare Masse = 18,02 g/mol |

In einem Reaktionsgefäß wurden 266,6 g (3-Aminopropyl)triethoxysilan (1,204 mol) vorgelegt. Unter Rühren wurden bei Raumtemperatur 933,4 g Methyltrimethoxysilan (6,852 mol) hinzugegeben. Im Anschluss daran wurden 133,4 g Wasser (7,403 mol) langsam unter Rühren hinzugetropft.

Das Molverhältnis (a3)/(a1) beträgt (7,403 mol) / (1,204 mol) = 6,15

Im Rahmen einer weiteren ganz besonders bevorzugten Ausführungsform ist eine kosmetische Zusammensetzung zur Behandlung von keratinischem Material dadurch gekennzeichnet, dass die organischen $C_1$-$C_6$-Alkoxy-Silane (a1) und Wasser (a3) zum Erhalt des Produktes (a) in einem Molverhältnis (a3)/(a1) von 4,0 bis 9,5, bevorzugt von 4,5 bis 9,0, weiter bevorzugt von 5,0 bis 8,5, noch weiter bevorzugt von 5,5 bis 8,0 und ganz besonders bevorzugt von 5,8 bis 6,4 miteinander vermischt werden.

Hierbei sind die organischen $C_1$-$C_6$-Alkoxy-Silane (a1) die zuvor beschriebenen organischen $C_1$-$C_6$-Alkoxy-Silane der Formel (I).

Auch die $C_1$-$C_6$-Alkoxy-Silane (a2) und Wasser (a3) werden bevorzugt in bestimmten Molverhältnissen miteinander zur Reaktion gebracht. Die besten Ergebnisse konnten erhalten werden, wenn die organischen $C_1$-$C_6$-Alkoxy-Silane (a2) und Wasser (a3) zum Erhalt des Produktes (a) in einem Molverhältnis (a3)/(a2) von 0,1 bis 3,5, bevorzugt von 0,3 bis 3,0, weiter bevorzugt von 0,5 bis 2,5, noch weiter bevorzugt von 0,7 bis 2,0 und ganz besonders bevorzugt von 0,9 bis 1,3 miteinander vermischt werden.

Bei Einhaltung dieser Molverhältnisse wird Wasser (a3) im Vergleich zu den $C_1$-$C_6$-Alkoxy-Silanen (a1) ganz besonders in ungefähr äquimolaren Mengen eingesetzt.

Beispiel:

| | | |
|---|---|---|
| (a1) | (3-Aminopropyl)triethoxysilan | molare Masse = 221,37 g/mol |
| (a2) | Methyltrimethoxysilan | molare Masse = 136,22 g/mol |
| (a3) | Wasser | molare Masse = 18,02 g/mol |

In einem Reaktionsgefäß wurden 266,6 g (3-Aminopropyl)triethoxysilan (1,204 mol) vorgelegt. Unter Rühren wurden bei Raumtemperatur 933,4 g Methyltrimethoxysilan (6,852 mol) hinzugegeben. Im Anschluss daran wurden 133,4 g Wasser (7,403 mol) langsam unter Rühren hinzugetropft.

Das Molverhältnis (a3)/(a2) beträgt (7,402 mol) / (6,852 mol) = 1,08

(3-Aminopropyl)triethoxysilan besitzt 3 hydrolysierbare Ethoxy-Gruppen pro Silan-Molekül. Methyltrimethoxysilan besitzt 3 hydrolysierbare Methoxy-Gruppen pro Silan-Molekül.

Summe der hydrolysierbaren $C_1$-$C_6$-Alkoxygruppen = [(1,204 mol) x 3] + [(6,852 mol) x 3] = 24,168 mol. Durch Zugabe von 133,4 g Wasser (7,403 mol) wurden 30,6 % (knapp ein Drittel) der in den Silanen vorhandenen $C_1$-$C_6$-Alkoxygruppen hydrolysiert.

Im Rahmen einer weiteren ganz besonders bevorzugten Ausführungsform ist eine kosmetische Zusammensetzung zur Behandlung von keratinischem Material dadurch gekennzeichnet, dass die organischen $C_1$-$C_6$-Alkoxy-Silane (a2) und (a3) Wasser zum Erhalt des Produktes (a) in einem Molverhältnis (a3)/(a2) von 0,1 bis 3,5, bevorzugt von 0,3 bis 3,0, weiter bevorzugt von 0,5 bis 2,5, noch weiter bevorzugt von 0,7 bis 2,0 und ganz besonders bevorzugt von 0,9 bis 1,3 miteinander vermischt werden.

Hierbei sind die organischen $C_1$-$C_6$-Alkoxy-Silane (a2) die zuvor beschriebenen organischen $C_1$-$C_6$-Alkoxy-Silane der Formel (II).

Die Herstellung des Produktes (a) aus den organischen $C_1$-$C_6$-Alkoxy-Silanen (a1) mit den organischen $C_1$-$C_6$-Alkoxy-Silanen (a2) und Wasser (a3) kann beispielsweise in einem Reaktionsgefäß oder einem Reaktor, bevorzugt in einem Doppelwandreaktor, einem Reaktor mit externem Wärmeaustauscher, einem Rohrreaktor, einem Reaktor mit Dünnfilm-Verdampfer, einem Reaktor mit Fallfilmverdampfer und/oder einem Reaktor mit angeschlossenem Kondensator durchgeführt werden.

Ein für kleinere Ansätze sehr gut geeignetes Reaktionsgefäß ist beispielsweise ein üblicherweise für chemische Reaktionen eingesetzter Glaskolben mit einem Fassungsvermögen von 1 Liter, 3 Litern oder 5 Litern handeln, beispielsweise ein 3-Liter Ein- oder Mehrhalskolben mit Schliffen.

Als Reaktor wird ein abgegrenzter Raum (Behältnis, Behälter) bezeichnet, der speziell dafür konstruiert und hergestellt wurde, um darin unter definierten Bedingungen bestimmte Reaktionen ablaufen lassen und steuern zu können.

Für größere Ansätze hat es sich als vorteilhaft herausgestellt, die Reaktion in Reaktoren aus Metall durchzuführen. Typische Reaktoren können beispielsweise eine Füllmenge von 10-Litern, 20-Litern oder 50-Litern umfassen. Größere Reaktoren für den Produktionsbereich können auch Füllmengen von 100-Litern, 500-Litern oder 1000-Litern umfassen.

Doppelwandreaktoren besitzen zwei Reaktor-Hüllen bzw. Reaktor-Wandungen, wobei in dem zwischen beiden Wandungen befindlichen Bereich eine Temperier-Flüssigkeit zirkulieren kann. Diese ermöglicht eine besonders gute Einstellung der Temperatur auf die benötigten Werte.

Als besonders gut geeignet hat sich auch der Einsatz von Reaktoren, insbesondere DoppelwandReaktoren mit vergrößerter Wärme-Austauschfläche erwiesen, wobei der Wärmeaustauch hierbei entweder durch interne Einbauten oder auch durch Einsatz eines externen Wärmeaustauschers erfolgen kann.

Entsprechende Reaktoren sind beispielsweise Laborreaktoren der Firma IKA. In diesem Zusammenhang können die Modelle "LR-2.ST" oder das Modell "magic plant" genannt werden.

Weitere einsetzbare Reaktoren sind Reaktoren mit Dünnfilm-Verdampfer, da auf diesem Wege eine sehr gute Wärmeabfuhr und damit eine besonders exakte Temperierung vorgenommen werden kann. Dünnfilm-Verdampfer werden alternativ auch als Dünnschicht-Verdampfer bezeichnet. Dünnschicht-Verdampfer können beispielsweise von der Asahi Glassplant Inc. kommerziell erworben werden.

Bei Reaktoren mit Fallfilmverdampfer findet die Verdampfung im Allgemeinen in einem Rohr statt, d.h. die zu verdampfende Flüssigkeit (d.h. in diesem Fall die in Schritt (2) zu entfernenden $C_1$-$C_6$-Alkohole) fließen als zusammenhängender Flüssigkeitsfilm. Auch Reaktoren mit Fallfilmverdampfer sind von verschiedenen Anbietern kommerziell erhältlich.

Für die Herstellung besonders leistungsstarker Keratinbehandlungsmittel hat sich bei der Herstellung des Produktes (a) die Einhaltung spezieller Temperaturbereiche als ganz besonders vorteilhaft herausgestellt.

In diesem Zusammenhang konnte gefunden werden, dass eine Mindest-Temperatur von 20 °C in Schritt (1) besonders gut geeignet ist, um die Hydrolyse mit ausreichend hoher Geschwindigkeit ablaufen zu lassen und eine effiziente Reaktionsführung zu gewährleisten.

Auf der anderen Seite sollte jedoch eine Erwärmung des Reaktionsgemisches auf Temperaturen über 70 °C bevorzugt vermieden werden. Erfolgt die Herstellung bei zu hohen Temperaturen, so findet vermutlich eine an dieser Stelle unerwünschte oder zu starke Polymerisierung bzw. Kondensationsreaktion statt, die dazu führt, dass sich bei der späteren Anwendung des Mittels auf dem Keratinmaterial kein an dem Keratin anhaftender Film mehr ausbilden kann. Bei Anwendung eines bei zu hohen Temperaturen hergestelltes Mittels in einem Färbeverfahren konnten somit keine ausreichend hohen Farbintensitäten mehr erzielt werden.

Aus diesen Gründen wird die Reaktion bzw. das Vermischen der organischen $C_1$-$C_6$-Alkoxy-Silanen (a1) mit den organischen $C_1$-$C_6$-Alkoxy-Silanen (a2) und Wasser (a3) bevorzugt bei einer Temperatur von 20 bis 70 °C durchgeführt.

Der hier angegebene Temperaturbereich bezieht sich auf die Temperatur, auf die das Gemisch aus $C_1$-$C_6$-Alkoxy-Silane (a1) und (a2) und Wasser (a3) eingestellt sein sollte. Gemessen werden kann diese Temperatur beispielsweise durch ein in diese Mischung hineinragendes, geeichtes Thermometer. Bevorzugt erfolgt die Reaktion eines oder mehrerer organischer $C_1$-$C_6$-Alkoxy-Silane mit Wasser bei einer Temperatur von 20 °C bis 70 °C, bevorzugt von 20 bis 65 °C, weiter bevorzugt von 20 bis 60 °C, noch weiter bevorzugt von 20 bis 55 °C, noch weiter bevorzugt von 20 bis 50 °C und ganz besonders bevorzugt von 20 bis 45 °C.

In einer weiteren Ausführungsform besonders bevorzugt ist eine kosmetische Zusammensetzung zur Behandlung von keratinischem Material, insbesondere keratinischen Fasern, enthaltend

(a) ein Produkt, das erhalten wird durch Mischen von

(a1) einem oder mehreren organischen $C_1$-$C_6$-Alkoxy-Silanen der Formel (I)

$$H_2N\text{-}L\text{-}Si(OR_1)_3 \qquad (I)$$

wobei

- L für eine lineare oder verzweigte, zweiwertige $C_1$-$C_{20}$-Alkylengruppe steht,
- $R_1$ für eine $C_1$-$C_6$-Alkylgruppe steht,
  mit

(a2) einem oder mehreren organischen $C_1$-$C_6$-Alkoxy-Silanen der Formel (II)

$$R_2\text{-}Si(OR_3)_3 \qquad (II)$$

wobei

- $R_2$ für eine $C_1$-$C_{12}$-Alkylgruppe steht, und
- R3 für eine $C_1$-$C_6$-Alkylgruppe steht, und

(a3) Wasser,

bei einer Temperatur von 20 °C bis 70 °C, bevorzugt von 20 bis 65 °C, weiter bevorzugt von 20 bis 60 °C, noch weiter bevorzugt von 20 bis 55 °C, noch weiter bevorzugt von 20 bis 50 °C und ganz besonders bevorzugt von 20 bis 45 °C.

Die Einstellung der bevorzugten und besonders bevorzugten Temperaturbereiche kann durch Temperieren des Reaktionsgefäßes oder Reaktors erfolgen. Beispielsweise kann das Reaktionsgefäß oder der Reaktor von außen

mit einem Temperierbad umgeben werden, bei dem es sich beispielsweise um ein Wasserbad oder um Silikonölbad handeln kann.

Wird die Reaktion in einem Doppelwandreaktor durchgeführt, so kann auch eine temperierte Flüssigkeit durch den Raum geleitet werden, der durch die beiden Wandungen gebildet wird und der den Reaktionsraum umgibt.

Es kann ferner bevorzugt sein, dass kein aktives Erwärmen der Reaktionsmischung erfolgt und eine etwaige Erhöhung der Temperatur über die Umgebungstemperatur nur durch die Exothermie der Hydrolyse bewirkt wird. Erwärmt der exotherme Reaktionsablauf die Reaktionsmischung stark, muss wiederum gekühlt werden.

Die Reaktion der organischer $C_1$-$C_6$-Alkoxy-Silane mit Wasser findet bevorzugt bei Normaldruck, d.h. bei einem Druck von 1013 mbar (1013 hPa) statt.

Die erfindungsgemäße Zusammensetzungen kann das Produkt (a) in verschiedenen Mengenanteilen enthalten. Diese bestimmt der Fachmann in Abhängigkeit von der gewünschten Dicke des Silan-Coatings auf dem Keratinmaterial.

Besonders lagerstabile Zusammensetzungen konnten erhalten werden, wenn die Zusammensetzung - bezogen auf ihr Gesamtgewicht - ein oder mehrere Produkte (a) in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-% enthält.

In einer weiteren Ausführungsform ganz besonders bevorzugt ist eine kosmetische Zusammensetzung zur Behandlung von keratinischem Material, enthaltend - bezogen auf das Gesamtgewicht der Zusammensetzung - ein oder mehrere Produkte (a) in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-%.

Gehalt von $C_1$-$C_6$-Alkoholen (b) in der Zusammensetzung

Wie bereits zuvor beschrieben wird durch das Vermischen der reaktiven $C_1$-$C_6$-Alkoxy-Silane (a1) und (a2) mit Wasser (a3) eine Hydrolyse-Reaktion initiiert, bei der die direkt am Silicium-Atom befindlichen $C_1$-$C_6$-Alkoxygruppen hydrolysiert und die entsprechenden $C_1$-$C_6$-Alkohole freigesetzt werden.

Auch die bei der Hydrolyse entstehenden partiell oder vollständig hydrolysierten Silane sind reaktive Verbindungen, die Folgereaktionen eingehen können, bei welchen diese Silane unterschiedlichen Hydrolysegrades miteinander kondensieren.

Es wird vermutet, dass unter anderem die folgenden Kondensationsreaktionen ablaufen können (mögliche Kondensationsreaktionen gezeigt anhand des Gemisches (3-Aminopropyl)triethoxysilan und Methyltrimethoxysilan):

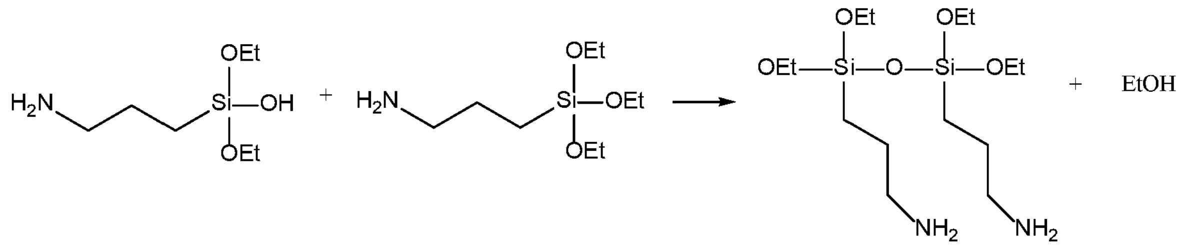

und/oder

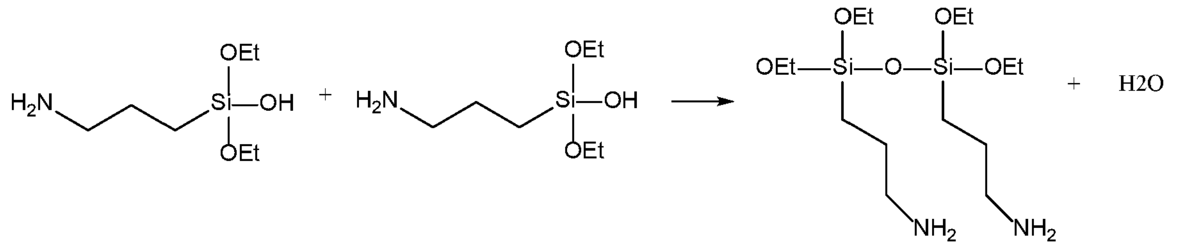

und/oder

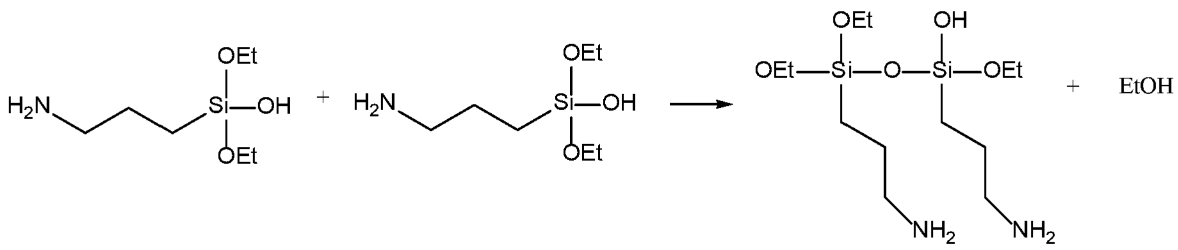

und/oder

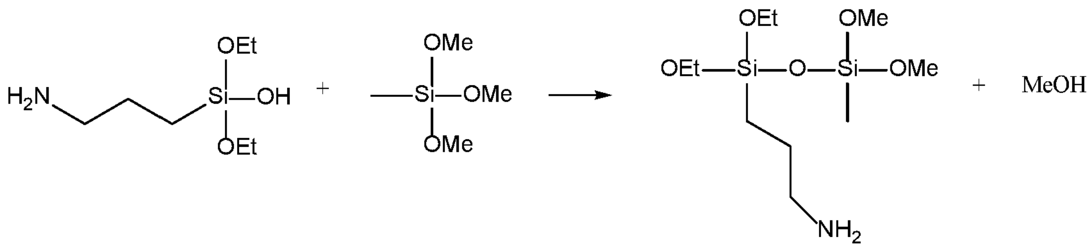

und/oder

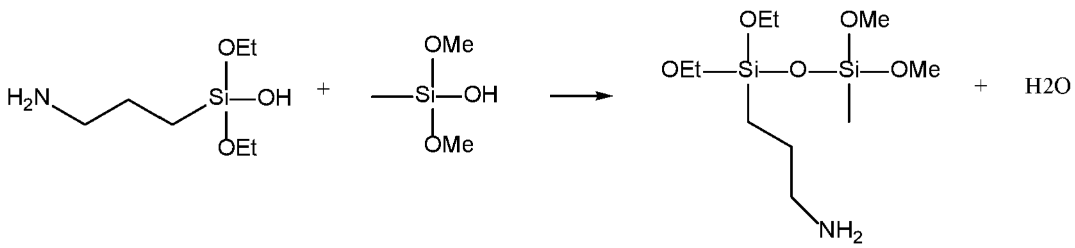

und/oder

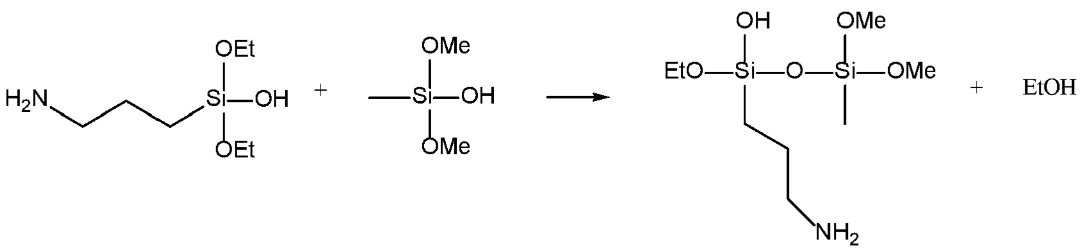

und/oder

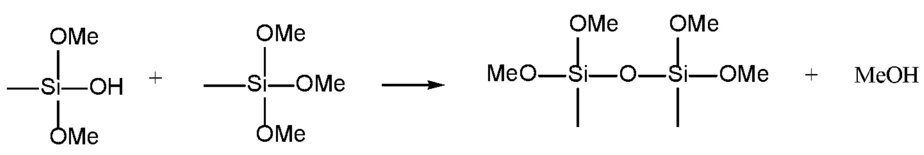

In den obigen beispielhaften Reaktionsschemata ist jeweils die Kondensation zu einem Dimer gezeigt, jedoch sind auch die weitergehende Kondensationen zu Oligomeren mit mehreren Silan-Atomen möglich und auch bevorzugt.

An diesen Kondensationsreaktionen können sowohl partiell hydrolysierte als auch vollständig hydrolysierte $C_1$-$C_6$-Alkoxysilane (a1) teilnehmen, die eine Kondensation mit noch nicht abreagierten, partiell oder auch vollständig hydrolysierten $C_1$-$C_6$-Alkoxysilanen (a1) durchlaufen. In diesem Fall reagieren die $C_1$-$C_6$-Alkoxysilane des Typs (a1) mit sich selbst.

Weiterhin können an den Kondensationsreaktionen auch partiell hydrolysierte als auch vollständig hydrolysierte $C_1$-$C_6$-Alkoxysilane (a2) teilnehmen, die eine Kondensation mit noch nicht abreagierten, partiell oder auch vollständig

hydrolysierten $C_1$-$C_6$-Alkoxysilanen (a1) durchlaufen. In diesem Fall reagieren die $C_1$-$C_6$-Alkoxysilane des Typs (a1) den $C_1$-$C_6$-Alkoxysilanen des Typs (a2).

Weiterhin können an den Kondensationsreaktionen auch partiell hydrolysierte als auch vollständig hydrolysierte $C_1$-$C_6$-Alkoxysilane (a2) teilnehmen, die eine Kondensation mit noch nicht abreagierten, partiell oder auch vollständig hydrolysierten $C_1$-$C_6$-Alkoxysilanen (a2) durchlaufen. In diesem Fall reagieren die $C_1$-$C_6$-Alkoxysilane des Typs (a2) mit sich selbst.

Vermutlich enthält das durch Vermischen von (a1), (a2) und (a3) hergestellte Produkt ein Gemisch all dieser dimeren und oligomeren Silan-Kondensate.

Wie sich aus den oben gezeigten Reaktionsschemata ablesen lässt, werden bei den Kondensationsreaktion wiederum entweder $C_1$-$C_6$-Alkohole (beispielsweise Ethanol und/oder Methanol) oder Wasser frei, wobei die Menge der frei werdenden $C_1$-$C_6$-Alkohole / Wasser davon abhängt, zu welchem Ausmaß das Gleichgewicht der obigen Reaktionen auf der Seite der Kondensate liegt.

Das Ausmaß der Kondensationsreaktion wiederum wird durch die anfangs zugegebene Menge an Wasser (a3) mitbestimmt. Bevorzugt wird die Menge an Wasser so bemessen, dass die Kondensation eine Teilkondensation ist, wobei "Teilkondensation" bzw. "partielle Kondensation" in diesem Kontext bedeutet, dass nicht alle kondensierbaren Gruppen der vorgelegten Silane miteinander reagieren, so dass die entstehende organische Siliciumverbindung pro Molekül im Mittel jeweils noch mindestens eine hydrolysierbare/kondensierbare Gruppe aufweist.

Die in der Kondensationsreaktion frei werdenden Mengen an $C_1$-$C_6$-Alkoholen und Wasser werden als Nebenprodukte betrachtet, deren Gehalt analytisch bestimmt werden kann und die durch verschiedene Trennmethoden (beispielsweise durch Destillation) aus dem Reaktionsgemisch entfernt werden können. Alle in der Zubereitung enthaltenen $C_1$-$C_6$-Alkohole und Wasser fallen daher nicht mit unter die Definition des Produktes (a). Mit dem Produkt (a) im Sinne der Erfindung ist ausschließlich das dimere bzw. oligomere Silan-Kondensat oder ein Gemisch entsprechender Silan-Kondensate gemeint.

Bei der Anwendung der erfindungsgemäßen Zubereitung auf dem Keratinmaterial ist die Erzeugung eines stabilen, zusammenhängenden und gleichmäßigen Coatings die Grundvoraussetzung für die Erzielung der gewünschten anwendungstechnischen Eigenschaften. Intensive und langanhaltende Färbungen können vor allem dann erhalten werden, wenn die farbgebenden Verbindungen in ein entsprechend resistentes Coating integriert werden können. Es hat sich herausgestellt, dass es hierfür wesentlich ist, den Gehalt an $C_1$-$C_6$-Alkoholen in der erfindungsgemäßen Zusammensetzung möglichst gering zu halten.

Aus diesem Grund besteht die Maßgabe, dass die erfindungsgemäße Zusammensetzung einen oder mehrere $C_1$-$C_6$-Alkohole (b) in einer Gesamtmenge von 0,001 bis 10,0 Gew.-% enthält.

Unter $C_1$-$C_6$-Alkoholen im Sinne der Erfindung werden Alkohole mit einer Hydroxygruppe oder mehreren Hydroxy-Gruppen verstanden, die 1 bis 6 Kohlenstoffatome umfassen. Diese Alkohole können linear oder verzweigt, gesättigt oder einfach bzw. mehrfach ungesättigt sein. Unter $C_1$-$C_6$-Mono-Alkoholen werden insbesondere die Alkohole aus der Gruppe aus Methanol, Ethanol, n-Propanol, Isopropanol, 1-Butanol, 2-Butanol, 1-Pentanol, 2-Pentanol, 3-Pentanol, 1-Hexanol, 2-Hexanol und 3-Hexanol verstanden. $C_1$-$C_6$-Alkohole mit zwei Hydroxygruppen sind beispielsweise Ethylenglycol, 1,2-Propandiol und 1,3-Propandiol. Ein $C_1$-$C_6$-Alkohol mit drei Hydroxygruppen ist beispielsweise Glycerin.

Zur Einhaltung dieser Gewichtsvorgaben müssen einerseits die $C_1$-$C_6$-Alkohole, die bei den zum Produkt (a) führenden Reaktionen frei werden, möglichst vollständig aus der Reaktionsgemisch entfernt werden. Darüber hinaus dürfen auch keine weiteren $C_1$-$C_6$-Alkohole in zu großen Mengen zur erfindungsgemäßen Zubereitung hinzugegeben werden.

Mit Zubereitungen, deren Gesamtgehalt an $C_1$-$C_6$-Alkoholen bei 10,0 Gew.-% lag, konnten bei Anwendung auf dem Keratinmaterial Färbungen mit ausreichend hoher Farbintensität erhalten werden.

Noch bessere Ergebnisse wurden jedoch erhalten, wenn der Gesamtgehalt an $C_1$-$C_6$-Alkoholen - bezogen auf das Gesamtgewicht der Zusammensetzung - auf eine Gesamtmenge von 0,01 bis 9,0 Gew.-%, bevorzugt von 0,1 bis 8,0 Gew.-%, weiter bevorzugt von 0,5 bis 7,0 Gew.-% und ganz besonders bevorzugt von 0,5 bis 4,0 Gew.-% beschränkt werden konnte.

In einer weiteren Ausführungsform ganz besonders bevorzugt ist eine Zusammensetzung, enthaltend - bezogen auf das Gesamtgewicht der Zusammensetzung -

(b) einen oder mehrere $C_1$-$C_6$-Alkohole in einer Gesamtmenge von 0,01 bis 9,0 Gew.-%, bevorzugt von 0,1 bis 8,0 Gew.-%, weiter bevorzugt von 0,5 bis 7,0 Gew.-% und ganz besonders bevorzugt von 0,5 bis 5,0 Gew.-% .

Hierbei erfolgt die Bestimmung bzw. Messung des Gehalts an $C_1$-$C_6$-Alkoholen bevorzugt innerhalb von 48 Stunden und ganz besonders bevorzugt innerhalb von 24 Stunden nach der Herstellung der Zubereitung, d.h. innerhalb von 48 bzw. 24 Stunden nach dem Erhalt des Produktes (a) durch Vermischen der drei Bestandteile (a1), (a2) und (a3).

Mit anderen Worten besonders bevorzugt ist eine Zusammensetzung, enthaltend - bezogen auf das Gesamtgewicht der Zusammensetzung -

(b) einen oder mehrere $C_1$-$C_6$-Alkohole in einer Gesamtmenge von 0,01 bis 10,0 Gew.-%, bevorzugt 0,01 bis 9,0 Gew.-

%, weiter bevorzugt von 0,1 bis 8,0 Gew.-%, noch weiter bevorzugt von 0,5 bis 7,0 Gew.-% und ganz besonders bevorzugt von 0,5 bis 5,0 Gew.-%, wobei die Bestimmung des Gehaltes an $C_1$-$C_6$-Alkoholen innerhalb von 24 Stunden nach dem Erhalt des Produktes (a) durch Vermischen der drei Bestandteile (a1), (a2) und (a3) erfolgt.

Die Bestimmung des Gehalts an $C_1$-$C_6$-Alkoholen in der erfindungsgemäßen Zubereitung kann mittels verschiedener analytischer Methoden erfolgen. Eine Möglichkeit ist die Messung mittels GC-MS. Die Gaschromatographie mit Massenspektrometrie-Kopplung ist die Kopplung eines Gas-Chromatographen (GC) mit einem Massenspektrometer (MS). Das Gesamtverfahren bzw. die Gerätekoppelung wird verkürzend auch als GC-MS, GC/MS oder GCMS bezeichnet

Zur Bestimmung des Gehaltes an $C_1$-$C_6$-Alkoholen kann eine Probe der Zubereitung beispielsweise in einer Doppelbestimmung auf einer unpolaren Säule gaschromatographisch untersucht werden. Die Identifizierung der zugeordneten Komponenten kann massenspektrometrisch über Bibliotheksvergleichsspektren (z.B. NIST oder Wiley) erfolgen. Aus den Doppelbestimmungen wird jeweils der Mittelwert gebildet. Quantifiziert werden kann beispielsweise mittels interner Standard-Kalibrierung (z.B. mit Methylisobutylketon).

Wie bereits beschrieben werden im erfindungsgemäßen Verfahren ganz besonders bevorzugt $C_1$-$C_6$-Alkoxysilane (a1) und (a2) eingesetzt, die Methoxysilan- oder Ethoxysilangruppen tragen. Diese haben den Vorteil, dass bei der Hydrolyse und Kondensation Methanol bzw. Ethanol freigesetzt werden, die aufgrund ihrer Siedepunkte leicht mittels Vakuumdestillation aus der Reaktionsmischung entfernt werden können.

Besonders bevorzugt wird in der erfindungsgemäßen Zusammensetzung der Methanolgehalt (b1) so weit wie möglich reduziert. Ein besonders gutes und resistentes Coating konnte erhalten werden, wenn die Zusammensetzung - bezogen ihr Gesamtgewicht - 0,01 bis 9,0 Gew.-%, bevorzugt 0,01 bis 8,0 Gew.-%, weiter bevorzugt von 0,01 bis 7,0 Gew.-% und ganz besonders bevorzugt von 0,01 bis 4,0 Gew.-% Methanol (b1) enthielt.

In einer weiteren Ausführungsform ganz besonders bevorzugt ist eine Zusammensetzung, enthaltend - bezogen auf das Gesamtgewicht der Zusammensetzung -

(b1) 0,01 bis 9,0 Gew.-%, bevorzugt 0,01 bis 8,0 Gew.-%, weiter bevorzugt von 0,01 bis 7,0 Gew.-% und ganz besonders bevorzugt von 0,01 bis 4,0 Gew.-% Methanol.

Die Messung des Methanolgehaltes in der erfindungsgemäßen Zusammensetzung erfolgt bevorzugt innerhalb von 48 Stunden und ganz besonders bevorzugt innerhalb von 24 Stunden nach der Herstellung der Zubereitung, d.h. innerhalb von 48 bzw. 24 Stunden nach dem Erhalt des Produktes (a) durch Vermischen der drei Bestandteile (a1), (a2) und (a3).

Mit anderen Worten besonders bevorzugt ist eine Zusammensetzung, enthaltend - bezogen auf das Gesamtgewicht der Zusammensetzung -

(b1) 0,01 bis 9,0 Gew.-%, bevorzugt 0,01 bis 8,0 Gew.-%, weiter bevorzugt von 0,01 bis 7,0 Gew.-% und ganz besonders bevorzugt von 0,01 bis 4,0 Gew.-% Methanol, wobei die Bestimmung des Gehaltes an Methanol innerhalb von 24 Stunden nach dem Erhalt des Produktes (a) durch Vermischen der drei Bestandteile (a1), (a2) und (a3) erfolgt.

Weiterhin besonders bevorzugt ist es auch, wenn der Ethanolgehalt (a2) in der erfindungsgemäßen Zusammensetzung innerhalb bestimmter Wertebereiche liegt. Ein besonders gutes und resistentes Coating konnte erhalten werden, wenn die Zusammensetzung - bezogen ihr Gesamtgewicht - 0,01 bis 9,0 Gew.-%, bevorzugt 0,1 bis 8,0 Gew.-%, weiter bevorzugt von 0,5 bis 7,0 Gew.-% und ganz besonders bevorzugt von 0,5 bis 4,0 Gew.-% Ethanol (b2) enthielt.

In einer weiteren Ausführungsform ganz besonders bevorzugt ist eine Zusammensetzung, enthaltend - bezogen auf das Gesamtgewicht der Zusammensetzung -

(b2) 0,01 bis 9,0 Gew.-%, bevorzugt 0,1 bis 8,0 Gew.-%, weiter bevorzugt von 0,5 bis 7,0 Gew.-% und ganz besonders bevorzugt von 0,5 bis 4,0 Gew.-% Ethanol.

Auch die Messung des Ethanolgehaltes in der erfindungsgemäßen Zusammensetzung erfolgt bevorzugt innerhalb von 48 Stunden und ganz besonders bevorzugt innerhalb von 24 Stunden nach der Herstellung der Zubereitung, d.h. innerhalb von 48 bzw. 24 Stunden nach dem Erhalt des Produktes (a) durch Vermischen der drei Bestandteile (a1), (a2) und (a3).

Mit anderen Worten besonders bevorzugt ist eine Zusammensetzung, enthaltend - bezogen auf das Gesamtgewicht der Zusammensetzung -

(b2) 0,01 bis 9,0 Gew.-%, bevorzugt 0,1 bis 8,0 Gew.-%, weiter bevorzugt von 0,5 bis 7,0 Gew.-% und ganz besonders bevorzugt von 0,5 bis 4,0 Gew.-% Ethanol, wobei die Bestimmung des Gehaltes an Ethanol innerhalb von 24 Stunden nach dem Erhalt des Produktes (a) durch Vermischen der drei Bestandteile (a1), (a2) und (a3) erfolgt.

Die Einhaltung der zuvor beschriebenen Maximalmengen an $C_1$-$C_6$-Alkoholen kann beispielsweise durch Entfernung der $C_1$-$C_6$-Alkohole aus dem Reaktionsgemisch erzielt werden. Eine besonders bevorzugte Methode zur Entfernung der $C_1$-$C_6$-Alkohole mittels Destillation.

In diesem Zusammenhang wird vermutet, dass zu heiße Temperaturen oberhalb von 70 °C die Kondensation in Richtung hochmolekularer Produkte verschieben, die zu groß sind, um sich bei der späteren Keratinbehandlung als geschlossener und resistenter Film auf dem Keratinmaterial abzulagern. Aus diesem Grund wird ist es bevorzugt, bei der Entfernung der $C_1$-$C_6$-Alkohole aus dem Reaktionsgemisch einen Temperaturbereich von 20 bis 70 °C einzuhalten.

Besonders bevorzugt ist es, bei der Entfernung freigesetzten $C_1$-$C_6$-Alkohole aus dem Reaktionsgemisch einen

Temperaturbereich von 20 bis 65 °C, bevorzugt von 20 bis 60 °C, weiter bevorzugt von 20 bis 55 °C, noch weiter bevorzugt von 20 bis 50 °C und ganz besonders bevorzugt von 20 bis 45 °C einzuhalten.

Der angegebene Temperaturbereich bezieht sich hierbei auf die Temperatur, auf die das Reaktionsgemisch eingestellt sein muss, während die $C_1$-$C_6$-Alkoxy-Silane aus dem Reaktionsgemisch entfernt werden. Gemessen werden kann auch diese Temperatur beispielsweise durch ein in diese Mischung hineinragendes, geeichtes Thermometer.

Bei der Entfernung der $C_1$-$C_6$-Alkohole kann die Einstellung der erfindungsgemäßen und bevorzugten Temperaturbereiche beispielsweise durch Erhitzen bzw, Kühlen des Reaktionsgefäßes oder Reaktors erfolgen, zum Beispiel durch Einsatz des Reaktionsgefäßes in einen Heizpilz, oder dadurch, dass das Reaktionsgefäß von außen mit einem temperierten Bad umgeben wird, bei dem es sich beispielsweise um ein Wasserbad oder um Silikonölbad handeln kann.

Wir die Reaktion in einem Doppelwandreaktor durchgeführt, so kann auch eine temperierte Flüssigkeit durch den Raum geleitet werden, der durch die beiden Wandungen gebildet wird und der den Reaktionsraum umgibt.

Um eine möglichst vollständige Entfernung der freigesetzten $C_1$-$C_6$-Alkohole ohne Überschreitung des erfindungswesentlichen Temperaturbereichs zu gewährleisten, werden die $C_1$-$C_6$-Alkohole bevorzugt unter (im Vergleich zum Normaldruck) reduziertem Druck entfernt. Als besonders vorteilhaft hat es sich in diesem Zusammenhang erwiesen die $C_1$-$C_6$-Alkohole mittels einem Destillationsaufsatz aus dem Reaktionsgemisch abzudestillieren. Bei dieser Destillation wird bevorzugt ein Druck von 10 bis 900 mbar, weiter bevorzugt von 10 bis 800 mbar, noch weiter bevorzugt von 10 bis 600 mbar und ganz besonders bevorzugt von 10 bis 300 mbar eingestellt.

Die Vakuumdestillation ist ein übliches chemisches Verfahren, für das die gängigen kommerziell erwerblichen Vakuumpumpen und Destillationsapparaturen eingesetzt werden können. Die Destillationsapparaturen können als Aufsatz auf dem Reaktionsgefäß oder Reaktor vorliegen.

Im Anschluss an die Vakuumdestillation können die flüchtigen Alkohole und ggf. auch mit abdestilliertes Wasser kondensiert und als flüssiges Destillat in einer Vorlage aufgefangen werden. Die Destillation kann optional unter Kühlung der verdampften Alkohole/Wasser mittels eines Kühlers erfolgen. Der reduzierte Druck kann mittels üblicher und im Stand der Technik bekannter Verfahren erzeugt werden, typischerweise mit einer Vakuumpumpe.

<u>Wassergehalt (c) in der Zubereitung</u>

Wie die zuvor gezeigten Reaktionsschemata zeigen, kann auch ein zu hoher Wassergehalt das Reaktionsgleichgewicht von der Seite der Silan-Kondensate zurück auf die Seite der monomeren Silane verschieben. Ohne auf diese Theorie beschränkt zu sein, wird in diesem Zusammenhang vermutet, dass vor allem die Anwesenheit einer ausreichend hohen Mengen an oligomeren Silan-Kondensaten wesentlich für die Erzielung eines gleichmäßigen und resistenten Coatings auf dem Keratinmaterial ist, welches wieder um die Grundvoraussetzung für die Erzeugung von Färbe-Ergebnissen mit ausreichend hoher Intensität ist.

Aus diesem Grund ist es erfindungswesentlich, den Wassergehalt in der erfindungsgemäßen Zusammensetzung auf einen Wert von 0,001 bis 10,0 Gew.-% Wasser (c) zu begrenzen.

Mit Zubereitungen, deren Wassergehalt (c) bei 10,0 Gew.-% lag, konnten bei Anwendung auf dem Keratinmaterial Färbungen mit ausreichend hoher Farbintensität erhalten werden.

Noch bessere Ergebnisse wurden jedoch erhalten, wenn die Zusammensetzung - bezogen auf das Gesamtgewicht der Zusammensetzung - 0,01 bis 9,0 Gew.-%, bevorzugt 0,1 bis 7,0 Gew.-%, weiter bevorzugt 0,2 bis 5,0 Gew.-% und ganz besonders bevorzugt 0,5 bis 3,0 Gew.-% Wasser (c) enthielt.

In einer weiteren Ausführungsform ganz besonders bevorzugt ist eine Zusammensetzung, enthaltend - bezogen auf das Gesamtgewicht der Zusammensetzung - (c) 0,01 bis 9,0 Gew.-%, bevorzugt 0,1 bis 7,0 Gew.-%, weiter bevorzugt 0,2 bis 5,0 Gew.-% und ganz besonders bevorzugt 0,5 bis 3,0 Gew.-% Wasser.

Die Messung des Wassergehalts in der erfindungsgemäßen Zusammensetzung erfolgt bevorzugt innerhalb von 48 Stunden und ganz besonders bevorzugt innerhalb von 24 Stunden nach der Herstellung der Zubereitung, d.h. innerhalb von 48 bzw. 24 Stunden nach dem Erhalt des Produktes (a) durch Vermischen der drei Bestandteile (a1), (a2) und (a3).

Mit anderen Worten besonders bevorzugt ist eine Zusammensetzung, enthaltend - bezogen auf das Gesamtgewicht der Zusammensetzung -

(c) 0,01 bis 9,0 Gew.-%, bevorzugt 0,1 bis 7,0 Gew.-%, weiter bevorzugt 0,2 bis 5,0 Gew.-% und ganz besonders bevorzugt 0,5 bis 3,0 Gew.-% Wasser, wobei die Bestimmung des Gehaltes an Wasser innerhalb von 24 Stunden nach dem Erhalt des Produktes (a) durch Vermischen der drei Bestandteile (a1), (a2) und (a3) erfolgt.

Die Bestimmung des Wassergehalts in der erfindungsgemäßen Zubereitung kann mittels verschiedener bekannter analytischer Methoden erfolgen. Eine Möglichkeit ist die Messung mittels GC-MS. Die Gaschromatographie mit Massenspektrometrie-Kopplung ist die Kopplung eines Gas-Chromatographen (GC) mit einem Massenspektrometer (MS). Das Gesamtverfahren bzw. die Gerätekoppelung wird verkürzend auch als GC-MS, GC/MS oder GCMS bezeichnet. Eine weitere Möglichkeit ist die Bestimmung des Wasserghaltes mittels Titration, beipsielsweise mittels Karl-Fischer Titration.

Eine weitere Möglichkeit ist die Bestimmung des Gehalts an Wasser über quantitative NMR-Spektren, insbesondere über quantitative $^{1}H$-NMR Spektren.

Weitere Inhaltsstoffe in der Zusammensetzung

Optional können die erfindungsgemäßen Zusammensetzungen auch einen oder mehrere weitere kosmetischer Inhaltsstoffe enthalten.

Bei den fakultativ in Schritt (3) einsatzbaren kosmetischen Inhaltsstoffen kann es sich um alle geeigneten Bestandteile handeln, um dem Mittel weitere positive Eigenschaften zu verleihen. Beispielsweise können in Schritt (3) des Verfahrens ein Lösungsmittel, ein verdickendes oder filmbildendes Polymer, eine oberflächenaktive Verbindung aus der Gruppe der nichtionischen, kationischen, anionischen oder zwitterionischen/amphoteren Tenside, der farbgebenden Verbindungen aus der Gruppe der Pigmente, der direktziehenden Farbstoffe, der Oxidationsfarbstoffvorprodukte, der Fettkomponenten aus der Gruppe der $C_8$-$C_{30}$-Fettalkohole, der Kohlenwasserstoffverbindungen, Fettsäureester, der zur Gruppe der pH-Regulatoren zugehörigen Säuren und Basen, der der Parfüme, der Konservierungsmittel, der Pflanzenextrakte und der Proteinhydrolysate handeln.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen.

Als ganz besonders bevorzugt hat es sich in diesem Zusammenhang erwiesen, in Schritt (3) einen kosmetischen Inhaltsstoff einzusetzen, welcher die Stabilität, insbesondere die Lagerstabilität des Keratinbehandlungsmittels weiter verbessert. In diesem Zusammenhang hat sich die Zugabe (3) eines oder mehrerer kosmetischer Inhaltsstoffe aus der Gruppe aus Hexamethyldisiloxan, Octamethyltrisiloxan, Decamethyltetrasiloxan, Hexamethylcyclotrisiloxan, Octamethylcyclotetrasiloxan und/oder Decamethylcyclopentasiloxan als besonders vorteilhaft im Hinblick auf die Erhöhung der Stabilität der Zusammensetzung erwiesen.

In einer weiteren ganz besonders bevorzugten Ausführungsform ist ein erfindungsgemäßes Verfahren gekennzeichnet durch die

(3) Zugabe eines oder mehrerer kosmetischer Inhaltsstoffe aus der Gruppe aus Hexamethyldisiloxan, Octamethyltrisiloxan, Decamethyltetrasiloxan, Hexamethylcyclotrisiloxan, Octamethylcyclotetrasiloxan und/oder Decamethylcyclopentasiloxan.

Hexamethyldisiloxan besitzt die CAS-Nummer 107-46-0 und kann beispielsweise bei Sigma-Aldrich kommerziell erworben werden.

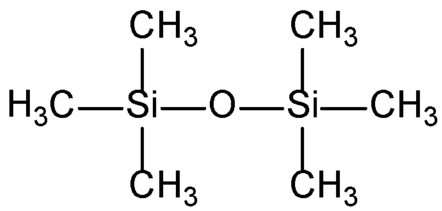

Octamethyltrisiloxan besitzt die CAS-Nummer 107-51-7 und ist ebenfalls bei Sigma-Aldrich kommerziell erhältlich.

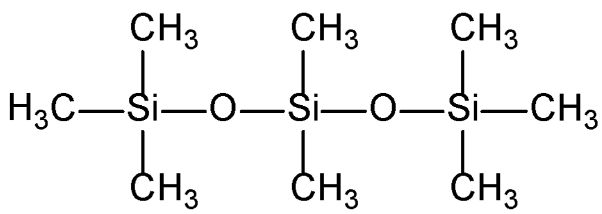

Decamethyltetrasiloxan trägt die CAS-Nummer 141-62-8 und ist ebenfalls bei Sigma-Aldrich kommerziell erhältlich.

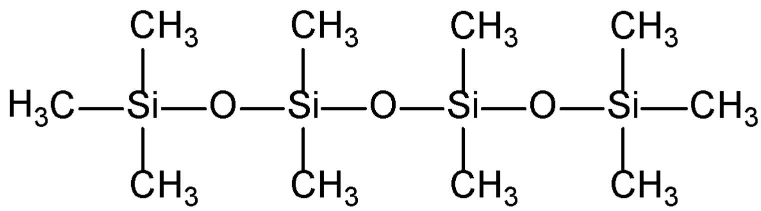

Hexamethylcyclotrisiloxan besitzt die CAS-Nr. 541-05-9.

Octamethylcyclotetrasiloxan besitzt die CAS-Nr. 556-67-2.

Decamethylcyclopentasiloxan besitzt die CAS-Nr. 541-02-6.

Um Zusammensetzungen mit besonders hoher Lagerstabilität zu erhalten, hat sich der Zusatz eines kosmetischen Inhaltsstoffes aus der Gruppe aus Hexamethyldisiloxan, Octamethyltrisiloxan, Decamethyltetrasiloxan, Hexamethylcyclotrisiloxan, Octamethylcyclotetrasiloxan und Decamethylcyclopentasiloxan als ganz besonders bevorzugt herausgestellt.

In einer weiteren Ausführungsform ganz besonders bevorzugt ist eine erfindungsgemäße Zusammensetzung, enthaltend

(d) einen oder mehrerere kosmetischer Inhaltsstoffe aus der Gruppe aus Hexamethyldisiloxan, Octamethyltrisiloxan, Decamethyltetrasiloxan, Hexamethylcyclotrisiloxan, Octamethylcyclotetrasiloxan und Decamethylcyclopentasiloxan.

Explizit ganz besonders bevorzugt ist der Einsatz von Hexamethyldisiloxan (d), da Zusammensetzungen mit diesem Inhaltsstoff sich als ganz besonders stabil erwiesen haben. Bevorzugt enthält die erfindungsgemäße Zusammensetzung (d) 10,0 bis 50,0 Gew.-%, bevorzugt 15,0 bis 45,0 Gew.-%, weiter bevorzugt 20,0 bis 40,0 Gew.-%, noch weiter bevorzugt 25,0 bis 35,0 Gew.-% und ganz besonders bevorzugt 31,0 bis 34,0 Gew.-% Hexamethyldisiloxan.

In einer weiteren Ausführungsform ganz besonders bevorzugt ist eine erfindungsgemäße Zusammensetzung, enthaltend - bezogen auf das Gesamtgewicht der Zusammensetzung - (d) 10,0 bis 50,0 Gew.-%, bevorzugt 15,0 bis 45,0 Gew.-%, weiter bevorzugt 20,0 bis 40,0 Gew.-%, noch weiter bevorzugt 25,0 bis 35,0 Gew.-% und ganz besonders bevorzugt 31,0 bis 34,0 Gew.-% Hexamethyldisiloxan.

pH-Werte der Zubereitungen

In weiteren Versuchen hat sich herausgestellt, dass auch der pH-Wert der erfindungsgemäßen Zusammensetzung einen Einfluss auf die Kondensationsreaktion nehmen kann. Hierbei wurde gefunden, dass insbesondere alkalische pH-Werte die Kondensation auf der Stufe der Oligomere anhalten. Je saurer das Reaktionsgemisch ist, desto schneller scheint die Kondensation abzulaufen und desto höher ist das Molekulargewicht der bei der Kondensation entstehenden Silan-Kondensate.

Aus diesem Grund ist es bevorzugt, wenn die Zusammensetzung einen pH-Wert von 7,0 bis 12,0, bevorzugt von 7,5 bis 11,5, weiter bevorzugt von 8,5 bis 11,0 und ganz besonders bevorzugt von 9,0 bis 11,0 besitzt.

Der Wassergehalt der Zusammensetzung liegt bei maximal 10,0 Gew.-% und wird bevorzugt noch geringer eingestellt. Insbesondere bei Zusammensetzungen mit sehr geringem Wassergehalt kann sich die Messung des pH-Wertes mit den üblichen aus dem Stand der Technik bekannten Methoden (pH-Wert Messung mittels Glaselektroden über Einstabmessketten oder aber über pH-Indikator-Papier) als schwierig erweisen. Aus diesem Grund handelt es sich bei den erfindungsgemäßen pH-Werten um die Werte, die nach Vermischen oder Verdünnung der Zubereitung im Gewichtsverhältnis 1:1 mit destilliertem Wasser erhalten wurden.

Der entsprechende pH-Wert wird dementsprechend gemessen, nachdem beispielsweise 50 g der erfindungsgemäßen Zusammensetzung mit 50 g destilliertem Wasser vermischt wurden.

In einer weiteren besonders bevorzugten Ausführungsform ist eine erfindungsgemäße Zusammensetzung dadurch gekennzeichnet, dass sie nach der Verdünnung mit destilliertem Wasser im Gewichtsverhältnis 1:1 einen pH-Wert von 7,0 bis 12,0, bevorzugt von 7,5 bis 11,5, weiter bevorzugt von 8,5 bis 11,0 und ganz besonders bevorzugt von 9,0 bis 11,0 besitzt.

Zur Einstellung dieses alkalischen pH-Wertes kann es erforderlich werden, der Reaktionsmischung ein Alkalisierungsmittel und/oder Acidifizierungsmittel zuzusetzen. Bei den pH-Werten im Sinne der vorliegenden Erfindung handelt es sich um pH-Werte, die bei einer Temperatur von 22°C gemessen wurden.

Als Alkalisierungsmittel können beispielsweise Ammoniak, Alkanolamine und/oder basische Aminosäuren eingesetzt werden.

Alkanolamine können ausgewählt werden aus primären Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol,

1-Amino-2-methyl-propan-2-ol, 3-Aminopropan-1,2-diol, 2-Amino-2-methylpropan-1,3-diol.

Als Aminosäure im Sinne der Erfindung gilt eine organische Verbindung, die in ihrer Struktur mindestens eine protonierbare Aminogruppe und mindestens eine -COOH- oder eine -SOsH-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus können auch anorganische Alkalisierungsmittel eingesetzt werden. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Neben den zuvor beschriebenen Alkalisierunsmitteln sind dem Fachmann zur Feineinstellung des pH-Wertes gängige Acidifizierungsmittel geläufig. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Mehrkomponenten-Verpackungseinheit (Kit-of-Parts)

Bei der zuvor beschriebenen Zusammensetzung handelt es sich um die lagerstabile Form des Silan-Blends (d.h. der Silan-Abmischung), der möglichst arm an $C_1$-$C_6$-Alkoholen ist und zudem einen besonders niedrigen Wassergehalt besitzt.

Für die Anwendung in einem Verfahren zur Behandlung von keratinischem Material, insbesondere zur Behandlung von keratinischen Fasern, muss der Anwender diesen lagerstabilen Blend in ein anwendungsbereites Mittel überführen. Das anwendungsbereite Mittel besitzt üblicherweise einen höheren Wassergehalt.

Zu diesem Zweck kann der Anwender kurz vor der Anwendung die zuvor beschriebene wasserarme Zusammensetzung (d.h. den Blend aus Silan-Kondensaten) mit einer oder mehreren weiteren Zusammensetzungen vermischen. Zur Erhöhung des Anwenderkomforts können dem Anwender alle benötigen Zusammensetzungen in Form einer Mehrkomponenten-Verpackungseinheit (Kit-of-Parts) zur Verfügung gestellt werden.

Explizit ganz besonders gute Eignung zeigen die Zusammensetzungen bei Anwendung in einem Färbeverfahren.

Bei Anwendung der erfindungsgemäßen Zubereitung in einem Färbeverfahren können eine oder mehrere farbgebende Verbindungen zum Einsatz kommen. Die farbgebenden Verbindung(en) können beispielsweise in einer separat konfektionierten kosmetischen Zubereitung (B) enthalten sein.

Ein zweiter Gegenstand der vorliegenden Erfindung ist eine Mehrkomponenten-Verpackungseinheit (Kit-of-parts) zum Färben von keratinischem Material, insbesondere menschlichen Haaren, welche getrennt voneinander konfektioniert

- eine erste Verpackungseinheit enthaltend eine kosmetische Zubereitung (A) und
- eine zweite Verpackungseinheit enthaltend eine kosmetische Zubereitung (B) umfasst,

wobei

- die kosmetische Zubereitung (A) eine Zusammensetzung ist, wie sie bei Beschreibung des ersten Erfindungsgegenstands im Detail offenbart wurde, und
- die kosmetische Zubereitung (B) mindestens eine farbgebende Verbindung aus der Gruppe der Pigmente und/oder

der direktziehenden Farbstoffe enthält.

Das oder die farbgebenden Verbindungen können vorzugsweise ausgewählt werden aus der Pigmente, der direktziehenden Farbstoffe, der Oxidationsfarbstoffe, der photochromen Farbstoffe und der thermochromen Farbstoffe, insbesondere bevorzugt aus Pigmenten und/oder direktziehenden Farbstoffen.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist en erfindungsgemäßes Mittel dadurch gekennzeichnet, dass es (b) mindestens eine farbgebende Verbindung aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte farbgebende Verbindungen aus der Gruppe der Pigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Mittel dadurch gekennzeichnet, dass es (b) mindestens eine farbgebende Verbindung aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbgebenden Verbindungen auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (b) mindestens eine farbgebende Verbindung enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona®, Colorona®, Xirona®, Dichrona® und Timiron® von der Firma Merck, Ariabel® und Unipure® von der Firma Sensient, Prestige® von der Firma Eckart Cosmetic Colors und Sunshine® von der Firma Sunstar erhältlich.

Ganz besonders bevorzugte Farbpigmente mit der Handelsbezeichnung Colorona® sind beispielsweise:

Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)

Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona® sind beispielsweise:

Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure® beispielsweise:

Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel auch (b) ein oder mehrere farbgebende Verbindungen aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einerweiteren besonders bevorzugten Ausführungsform ist ein erfindungsgemäßes Mittel dadurch gekennzeichnet, dass es (b) mindestens eine farbgebende Verbindungen aus der Gruppe der organischen Pigmente enthält,

die ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem erfindungsgemäßen Mitteln besonders bevorzugt. Weiterhin ist es bevorzugt, wenn die eingesetzten Pigmente eine bestimmte Teilchengröße aufweisen. Diese Teilchengröße führt einerseits zu einer gleichmäßigen Verteilung der Pigmente in dem gebildeten Polymerfilm und vermeidet andererseits ein raues Haar- oder Hautgefühl nach dem Auftragen des kosmetischen Mittels. Es ist daher erfindungsgemäß vorteilhaft, wenn das mindestens eine Pigment eine mittlere Teilchengröße $D_{50}$ von 1,0 bis 50 $\mu$m, vorzugsweise von 5,0 bis 45 $\mu$m, bevorzugt von 10 bis 40 $\mu$m, insbesondere von 14 bis 30 $\mu$m, aufweist. Die mittlere Teilchengröße $D_{50}$ kann beispielsweise unter Verwendung von dynamischer Lichtstreuung (DLS) bestimmt werden.

Das oder die Pigmente (b) können in einer Menge von 0,001 bis 20 Gew.-%, insbesondere von 0,05 bis 5 Gew.-%, jeweils bezogen auf das Gesamtgewicht des erfindungsgemäßen Mittels, eingesetzt werden.

Als farbgebende Verbindungen (b) können die erfindungsgemäßen Mittel auch einen oder mehrere direktziehende Farbstoffe enthalten. Bei direktziehende Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L. Besonders bevorzugt besitzen die direktziehenden Farbstoffe im Sinne der vorliegenden Erfindung eine Löslichkeit in Wasser (760 mmHg) bei 25 °C von mehr als 1,5 g/L.

Direktziehende Farbstoffe können in anionische, kationische und nichtionische direktziehende Farbstoffe unterteilt werden.

In einer weiteren bevorzugten Ausführungsform ist ein erfindungsgemäßes Mittel dadurch gekennzeichnet, dass es als farbgebende Verbindung (b) mindestens einen anionischen, kationischen und/oder nichtionischen direktziehenden Farbstoff enthält.

In einer weiteren bevorzugten Ausführungsform ist ein erfindungsgemäßes Mittel dadurch gekennzeichnet, dass es (b) mindestens einen anionischen, kationischen und/oder nichtionischen direktziehenden Farbstoff enthält.

Geeignete kationische direktziehende Farbstoffe sind beispielsweise Basic Blue 7, Basic Blue 26, Basic Violet 2 und Basic Violet 14, Basic Yellow 57, Basic Red 76, Basic Blue 16, Basic Blue 347 (Cationic Blue 347/ Dystar), HC Blue No. 16, Basic Blue 99, Basic Brown 16, Basic Brown 17, Basic Yellow 57, Basic Yellow 87, Basic Orange 31, Basic Red 51 Basic Red 76

Als nichtionische direktziehende Farbstoffe können beispielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)-amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-$SO_3$H) besitzen. In Abhängigkeit vom pH-Wert liegen die protonierten Formen (-COOH,

$-SO_3H$) der Carbonsäure- bzw. Sulfonsäuregruppierungen mit ihren deprotonierten Formen ($-COO^-$, $-SO_3^-$ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Als besonders gut geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, Iodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1 ,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%.

Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

Weiterhin können auch thermochrome Farbstoffe eingesetzt werden. Thermochromie beinhaltet die Eigenschaft eines Materials, in Abhängigkeit der Temperatur reversibel oder irreversibel seine Farbe zu ändern. Dies kann sowohl durch Änderung der Intensität und/oder des Wellenlängenmaximums erfolgen.

Schließlich ist es auch möglich, photochrome Farbstoffe einzusetzen. Photochromie beinhaltet die Eigenschaft eines Materials, in Abhängigkeit der Bestrahlung mit Licht, insbesondere UV-Licht, reversibel oder irreversibel seine Farbe zu ändern. Dies kann sowohl durch Änderung der Intensität und/oder des Wellenlängenmaximums erfolgen.

Die kosmetische Zubereitung (B) kann - bezogen auf das Gesamtgewicht der kosmetische Zubereitung (B) - ein oder mehrere Pigmente in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthalten.

Die kosmetische Zubereitung (B) kann - bezogen auf das Gesamtgewicht der kosmetische Zubereitung (B) - ein oder mehrere direktziehende Farbstoffe in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthalten.

Neben den Zubereitungen (A) und (B) kann die erfindungsgemäße Mehrkomponenten-Verpackungseinheit (Kit-of-parts) auch noch einen oder mehrere weitere getrennt konfektionierte Zubereitungen enthalten, beispielsweise eine kosmetische Zubereitung (C), die mindestens ein verdickendes Polymer enthält, und/oder eine kosmetische Zubereitung (D), die mindestens ein filmbildendes Polymer enthält,.

Im Rahmen einer weiteren Ausführungsform ganz besonders bevorzugt ist eine Mehrkomponenten-Verpackungseinheit (Kit-of-parts), umfassend

- eine dritte Verpackungseinheit enthaltend eine kosmetische Zubereitung (C), wobei die kosmetische Zubereitung (C) mindestens ein verdickendes Polymer enthält

Als verdickendes Polymer kann beispielsweise eingesetzt werden:

- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) und Natrosol®-Typen (Hercules) vertrieben werden.
- Stärke und deren Derivate, insbesondere Stärkeether, beispielsweise Structure® XL (National Starch), eine multifunktionelle, salztolerante Stärke;
- Polyvinylpyrrolidone, wie sie beispielsweise unter der Bezeichnung Luviskol® (BASF) vertrieben werden.

Im Rahmen einer weiteren Ausführungsform ganz besonders bevorzugt ist eine Mehrkomponenten-Verpackungseinheit (Kit-of-parts), umfassend

- eine vierte Verpackungseinheit enthaltend eine kosmetische Zubereitung (D), wobei die kosmetische Zubereitung (D) mindestens ein filmbildendes Polymer enthält

Als filmbildendes Polymer kann besonders bevorzugt mindestens ein anionisches Polymer aus der Gruppe der Copolymere von Acrylsäure, der Copolymere der Methacrylsäure, der Homopolymere oder Copolymere von Acrylsäure-Estern, der Homopolymere oder Copolymere von MethacrylsäureEstern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere

des Ethylens, der Homopolymere oder Copolymere des Propylens, der Homopolymere oder Copolymere des Styrens, der Polyurethane, der Polyester und/oder der Polyamide eingesetzt werden.

Betreffend die weiteren bevorzugten Ausführungsformen der erfindungsgemäßen Mehrkomponenten-Verpackungseinheit gilt *mutatis mutantis* das zum erfindungsgemäßen Zusammensetzungen gesagte.

Beispiele

1. Herstellung der Silan-Blends

1.1. Herstellung des Silan-Blends 1 (Vergleich)

Ein Reaktor mit beheizbarer/kühlbarer Außenhülle und mit einem Fassungsvermögen von 10 Litern wurde mit 4,67 kg Methyltrimethoxysilan (34,283 mol) befüllt. Unter Rühren wurden dann 1,33 kg (3-Aminopropyl)triethoxysilan (6,008 mol) hinzugegeben. Dieses Gemisch wurde bei 30 °C gerührt. Im Anschluss daran wurden 670 ml destilliertes Wasser (37,18 mol) tropfenweise unter kräftigem Rühren hinzugegeben, wobei die Temperatur des Reaktionsgemisches unter externer Kühlung bei 30 °C gehalten wurde. Nach Beendigung der Wasserzugabe wurde für weitere 10 Minuten nachgerührt.

| | | |
|---|---|---|
| (a1) | 3-(Triethoxysilyl)propylamin | molare Masse = 221,37 g/mol |
| (a2) | Methyltrimethoxysilan | molare Masse = 136,22 g/mol |
| (a3) | Wasser | molare Masse = 18,02 g/mol |

$$\text{Molverhältnis (a2)/(a1)} = 34{,}283\ \text{mol} / 6{,}008\ \text{mol} = 5{,}706$$

$$\text{Molverhältnis (a3)/(a1)} = 37{,}189\ \text{mol} / 6{,}008\ \text{mol} = 6{,}18$$

$$\text{Molverhältnis (a3)/(a2)} = 37{,}18\ \text{mol} / 34{,}283 = 1{,}08$$

Danach wurde ein Vakuum von 700 mbar angelegt und das Reaktionsgemisch auf eine Temperatur von 44 °C erwärmt. Sobald das Reaktionsgemisch die Temperatur von 44 °C erreicht hatte, wurden das bei der Reaktion freigesetzte Ethanol und Methanol über einen Zeitraum von 40 Minuten abdestilliert. Die abdestillierten Alkohole wurden in einer gekühlten Vorlage aufgefangen. Danach ließ man das Reaktionsgemisch auf Raumtemperatur abkühlen. Zu dem auf diese Weise erhaltenen Gemisch wurden dann unter Rühren 3,33 kg Hexamethyldisiloxan getropft. Es wurde 10 Minuten nachgerührt. Jeweils 100 ml des Silan-Blends wurden in eine Flasche mit 100 ml Fassungsvermögen und Schraubdeckelverschluss mit Dichtung abgefüllt. Nach dem Abfüllen wurden die Flaschen fest verschlossen. Der Wassergehalt betrug kleiner 2,0 Gew.-%.

1.2. Herstellung des Silan-Blends (2, Erfindung)

Ein Reaktor mit beheizbarer/kühlbarer Außenhülle und mit einem Fassungsvermögen von 10 Litern wurde mit 4,67 kg Methyltrimethoxysilan (34,283 mol) befüllt. Unter Rühren wurden dann 1,33 kg (3-Aminopropyl)triethoxysilan (6,008 mol) hinzugegeben. Dieses Gemisch wurde bei 30 °C gerührt. Im Anschluss daran wurden 670 ml destilliertes Wasser (37,18 mol) tropfenweise unter kräftigem Rühren hinzugegeben, wobei die Temperatur des Reaktionsgemisches unter externer Kühlung bei 30 °C gehalten wurde. Nach Beendigung der Wasserzugabe wurde für weitere 10 Minuten nachgerührt.

| | | |
|---|---|---|
| (a1) | 3-(Triethoxysilyl)propylamin | molare Masse = 221,37 g/mol |
| (a2) | Methyltrimethoxysilan | molare Masse = 136,22 g/mol |
| (a3) | Wasser | molare Masse = 18,02 g/mol |

$$\text{Molverhältnis (a2)/(a1)} = 34{,}283\ \text{mol} / 6{,}008\ \text{mol} = 5{,}706$$

$$\text{Molverhältnis (a3)/(a1)} = 37{,}189 \text{ mol} / 6{,}008 \text{ mol} = 6{,}18$$

$$\text{Molverhältnis (a3)/(a2)} = 37{,}18 \text{ mol} / 34{,}283 = 1{,}08$$

Danach wurde ein Vakuum von 280 mbar angelegt und das Reaktionsgemisch auf eine Temperatur von 44 °C erwärmt. Sobald das Reaktionsgemisch die Temperatur von 44 °C erreicht hatte, wurden das bei der Reaktion freigesetzte Ethanol und Methanol über einen Zeitraum von 190 Minuten abdestilliert. Im Verlauf der Destillation wurde das Vakuum auf 200 mbar abgesenkt. Die abdestillierten Alkohole wurden in einer gekühlten Vorlage aufgefangen. Danach ließ man das Reaktionsgemisch auf Raumtemperatur abkühlen. Zu dem auf diese Weise erhaltenen Gemisch wurden dann unter Rühren 3,33 kg Hexamethyldisiloxan getropft. Es wurde 10 Minuten nachgerührt. Jeweils 100 ml des Silan-Blends wurden in eine Flasche mit 100 ml Fassungsvermögen und Schraubdeckelverschluss mit Dichtung abgefüllt. Nach dem Abfüllen wurden die Flaschen fest verschlossen. Der Wassergehalt betrug kleiner 2,0 Gew.-%.

2. Messung des Gehaltes an $C_1$-$C_6$-Alkoholen mittels GC-MS

Innerhalb von 24 Stunden nach ihrer Abfüllung wurde von beiden Silan-Blends der Gehalt an Ethanol und an Methanol mittels GC-MS Spektroskopie gemessen. In einer Doppelbestimmung wurde jeweils eine Probe des Silan-Blends auf einer unpolaren Säule gaschromatographisch untersucht. Die Identifizierung der zugeordneten Komponenten erfolgte massenspektrometrisch über Bibliotheksvergleichsspektren (NIST /Wiley). Die Quantifizierung erfolgte mittels interner Standard-Kalibrierung mit Methylisobutylketon. Aus jeder Doppelbestimmung wurde jeweils der Mittelwert gebildet.

| | Silan-Blend 1 (Vergleich) | Silan-Blend 2 (Erfindung) |
|---|---|---|
| Methanol (Gew.-%) | 10,6 / 11,6 | 2,9 / 2,5 |
| Ethanol (Gew.-%) | 4,6 / 5,3 | 1,5 / 1,4 |
| Summe Gehalt $C_1$-$C_6$-Alkohole (Mittelwert) | 16,1 | 4,2 |

3. Färbung

Es wurde das folgende Färbemittel bereitgestellt (Zubereitung (B)).

Zubereitung (B)

| | |
|---|---|
| Colorona Bordeaux, Merck, MICA, CI 77491 (IRON OXIDES) | 5,5 g |
| Eco Smooth Satin (Ethylene/Sodium Acrylate Copolymer 25%ige Lösung, Dow Chemical Company) | 40,0 |
| Wasser | Ad 100 g |

Aus den Flaschen mit dem zuvor hergestellten Silan-Blend wurden jeweils 20 g abgewogen (Zubereitung A). Das anwendungsbereite Färbemittel wurde jeweils durch Verschütteln von 20 g der Zubereitung (A) und 100 g der Zubereitung (B) hergestellt (Schütteln für 3 Minuten). Dieses Gemisch wurde dann für 5 Minuten stehen gelassen.

Für die Anwendung wurde jeweils eine Haarsträhne (Kerling Euronaturhaar weiß) in das anwendungsbereite Färbemittel getaucht und für 1 Minute darin belassen. Danach wurde überflüssiges Mittel von jeder Haarsträhne gestreift. Dann wurde jede Haarsträhne mit Wasser ausgewaschen und getrocknet. Im Anschluss daran wurden die Strähnen visuell unter einer Tageslichtlampe bewertet. Hierbei wurden die folgenden Ergebnisse erhalten:

| Silan-Blend 1<br>Vergleich<br>20 g | Silan-Blend 2<br>Erfindung<br>20 g |
|---|---|
| Färbemittel (B) | Färbemittel (B) |

(fortgesetzt)

| | |
|---|---|
| 100 g | 100 g |
| Färbung: bordeaux-rot | Färbung: bordeaux-rot |
| Farbintensität: niedrig | Farbintensität: hoch |
| Deckvermögen: mittel | Deckvermögen: mittel |

## Patentansprüche

**1.** Kosmetische Zusammensetzung zur Behandlung von keratinischem Material, insbesondere keratinischen Fasern, enthaltend

(a) ein Produkt, das erhalten wird durch Mischen von

(a1) einem oder mehreren organischen $C_1$-$C_6$-Alkoxy-Silanen der Formel (I)

$$H_2N\text{-}L\text{-}Si(OR_1)_3 \qquad (I)$$

wobei

- L für eine lineare oder verzweigte, zweiwertige $C_1$-$C_{20}$-Alkylengruppe steht,
- $R_1$ für eine $C_1$-$C_6$-Alkylgruppe steht,

mit

(a2) einem oder mehreren organischen $C_1$-$C_6$-Alkoxy-Silanen der Formel (II)

$$R_2\text{-}Si(OR_3)_3 \qquad (II)$$

wobei

- $R_2$ für eine $C_1$-$C_{12}$-Alkylgruppe steht, und
- R3 für eine $C_1$-$C_6$-Alkylgruppe steht, und

(a3) Wasser, und - bezogen auf das Gesamtgewicht der Zusammensetzung -

(b) einen oder mehrere $C_1$-$C_6$-Alkohole in einer Gesamtmenge von 0,001 bis 10,0 Gew.-% und
(c) 0,001 bis 10,0 Gew.-% Wasser.

**2.** Zusammensetzung nach Anspruch 1, enthaltend

(a) ein Produkt, das erhalten wird durch Mischen von

(a1) (3-Aminopropyl)triethoxysilan mit
(a2) Methyltrimethoxysilan und
(a3) Wasser.

**3.** Zusammensetzung nach Anspruch 1, enthaltend

(a) ein Produkt, das erhalten wird durch Mischen von

(a1) (3-Aminopropyl)triethoxysilan mit
(a2) Ethyltriethoxysilan und
(a3) Wasser.

**4.** Zusammensetzung nach Anspruch 1, enthaltend

(a) ein Produkt, das erhalten wird durch Mischen von

(a1) (3-Aminopropyl)triethoxysilan mit
(a2) Methyltriethoxysilan und
(a3) Wasser.

**5.** Zusammensetzung nach Anspruch 1, enthaltend

(a) ein Produkt, das erhalten wird durch Mischen von

(a1) (3-Aminopropyl)triethoxysilan mit
(a2) Propyltriethoxysilan und
(a3) Wasser.

**6.** Zusammensetzung nach Anspruch 1, enthaltend

(a) ein Produkt, das erhalten wird durch Mischen von

(a1) (3-Aminopropyl)triethoxysilan mit
(a2) Hexyltriethoxysilan und
(a3) Wasser.

**7.** Zusammensetzung nach Anspruch 1, enthaltend

(a) ein Produkt, das erhalten wird durch Mischen von

(a1) (3-Aminopropyl)triethoxysilan mit
(a2) Octyltriethoxysilan und
(a3) Wasser.

**8.** Zusammensetzung nach einem der Ansprüche 1 bis 7, **dadurch gekennzeichnet, dass** die organischen $C_1$-$C_6$-Alkoxy-Silane (a1) und die organischen $C_1$-$C_6$-Alkoxy-Silane (a2) zum Erhalt des Produktes (a) in einem Molverhältnis (a2)/(a1) von 3,0 bis 8,0, bevorzugt von 3,5 bis 7,5, weiter bevorzugt von 4,0 bis 7,0, noch weiter bevorzugt von 4,5 bis 6,5 und ganz besonders bevorzugt von 5,4 bis 5,9 miteinander vermischt werden.

**9.** Zusammensetzung nach einem der Ansprüche 1 bis 8, **dadurch gekennzeichnet, dass** die organischen $C_1$-$C_6$-Alkoxy-Silane (a1) und Wasser (a3) zum Erhalt des Produktes (a) in einem Molverhältnis (a3)/(a1) von 4,0 bis 9,5, bevorzugt von 4,5 bis 9,0, weiter bevorzugt von 5,0 bis 8,5, noch weiter bevorzugt von 5,5 bis 8,0 und ganz besonders bevorzugt von 5,8 bis 6,4 miteinander vermischt werden.

**10.** Zusammensetzung nach einem der Ansprüche 1 bis 9, **dadurch gekennzeichnet, dass** die organischen $C_1$-$C_6$-Alkoxy-Silane (a2) und (a3) Wasser zum Erhalt des Produktes (a) in einem Molverhältnis (a3)/(a2) von 0,1 bis 3,5, bevorzugt von 0,3 bis 3,0, weiter bevorzugt von 0,5 bis 2,5, noch weiter bevorzugt von 0,7 bis 2,0 und ganz besonders bevorzugt von 0,9 bis 1,3 miteinander vermischt werden.

**11.** Zusammensetzung nach einem der Ansprüche 1 bis 10, enthaltend - bezogen auf das Gesamtgewicht der Zusammensetzung - ein oder mehrere Produkte (a) in einer Gesamtmenge von 30,0 bis 85,0 Gew.-%, bevorzugt von 35,0 bis 80,0 Gew.-%, weiter bevorzugt von 40,0 bis 75,0 Gew.-%, noch weiter bevorzugt von 45,0 bis 70,0 Gew.-% und ganz besonders bevorzugt von 50,0 bis 65,0 Gew.-%.

**12.** Zusammensetzung nach einem der Ansprüche 1 bis 11, enthaltend - bezogen auf das Gesamtgewicht der Zusammensetzung -
(b) einen oder mehrere $C_1$-$C_6$-Alkohole in einer Gesamtmenge von 0,01 bis 9,0 Gew.-%, bevorzugt von 0,1 bis 8,0 Gew.-%, weiter bevorzugt von 0,5 bis 7,0 Gew.-% und ganz besonders bevorzugt von 0,5 bis 5,0 Gew.-% .

**13.** Zusammensetzung nach einem der Ansprüche 1 bis 12, enthaltend - bezogen auf das Gesamtgewicht der Zusammensetzung - (b1) 0,01 bis 9,0 Gew.-%, bevorzugt 0,01 bis 8,0 Gew.-%, weiter bevorzugt von 0,01 bis 7,0 Gew.-% und ganz besonders bevorzugt von 0,01 bis 4,0 Gew.-% Methanol.

**14.** Zusammensetzung nach einem der Ansprüche 1 bis 13, enthaltend - bezogen auf das Gesamtgewicht der Zusammensetzung - (b2) 0,01 bis 9,0 Gew.-%, bevorzugt 0,1 bis 8,0 Gew.-%, weiter bevorzugt von 0,5 bis 7,0 Gew.-% und ganz besonders bevorzugt von 0,5 bis 4,0 Gew.-% Ethanol.

**15.** Zusammensetzung nach einem der Ansprüche 1 bis 14, enthaltend - bezogen auf das Gesamtgewicht der Zusammensetzung -
(c) 0,01 bis 9,0 Gew.-%, bevorzugt 0,1 bis 7,0 Gew.-%, weiter bevorzugt 0,2 bis 5,0 Gew.-% und ganz besonders bevorzugt 0,5 bis 3,0 Gew.-% Wasser.

**16.** Zusammensetzung nach einem der Ansprüche 1 bis 15, enthaltend
(d) einen oder mehrerere kosmetischer Inhaltsstoffe aus der Gruppe aus Hexamethyldisiloxan, Octamethyltrisiloxan, Decamethyltetrasiloxan, Hexamethylcyclotrisiloxan, Octamethylcyclotetrasiloxan und Decamethylcyclopentasiloxan.

**17.** Zusammensetzung nach einem der Ansprüche 1 bis 16, enthaltend - bezogen auf das Gesamtgewicht der Zusammensetzung - (d) 10,0 bis 50,0 Gew.-%, bevorzugt 15,0 bis 45,0 Gew.-%, weiter bevorzugt 20,0 bis 40,0 Gew.-%, noch weiter bevorzugt 25,0 bis 35,0 Gew.-% und ganz besonders bevorzugt 31,0 bis 34,0 Gew.-% Hexamethyldisiloxan.

**18.** Zusammensetzung nach einem der Ansprüche 1 bis 17, **dadurch gekennzeichnet, dass** sie nach der Verdünnung mit destilliertem Wasser im Gewichtsverhältnis 1:1 einen pH-Wert von 7,0 bis 12,0, bevorzugt von 7,5 bis 11,5, weiter bevorzugt von 8,5 bis 11,0 und ganz besonders bevorzugt von 9,0 bis 11,0 besitzt.

**19.** Mehrkomponenten-Verpackungseinheit (Kit-of-parts) zum Färben von keratinischem Material, insbesondere menschlichen Haaren, welche getrennt voneinander konfektioniert

- eine erste Verpackungseinheit enthaltend eine kosmetische Zubereitung (A) und
- eine zweite Verpackungseinheit enthaltend eine kosmetische Zubereitung (B) umfasst, wobei
- die kosmetische Zubereitung (A) eine Zusammensetzung nach einem der Ansprüche 1 bis 18 ist und
- die kosmetische Zubereitung (B) mindestens eine farbgebende Verbindung aus der Gruppe der Pigmente und/oder der direktziehenden Farbstoffe enthält.

**20.** Mehrkomponenten-Verpackungseinheit (Kit-of-parts) nach Anspruch 19, umfassend

- eine dritte Verpackungseinheit enthaltend eine kosmetische Zubereitung (C), wobei die kosmetische Zubereitung (C) mindestens ein verdickendes Polymer enthält

**21.** Mehrkomponenten-Verpackungseinheit (Kit-of-parts) nach einem der Ansprüche 19 bis 20, umfassend

- eine vierte Verpackungseinheit enthaltend eine kosmetische Zubereitung (D), wobei die kosmetische Zubereitung (D) mindestens ein filmbildendes Polymer enthält

## Claims

**1.** Cosmetic composition for the treatment of keratinous material, in particular keratinous fibers, comprising

(a) a product obtained by mixing

(a1) one or more organic $C_1$-$C_6$-alkoxy-silanes of the formula (I)

$$H_2N\text{-}L\text{-}Si(OR_1)_3 \qquad (I)$$

where

- L is a linear or branched divalent $C_1$-$C_{20}$-alkylene group,
- $R_1$ represents a $C_1$-$C_6$ alkyl-group,

with
(a2) one or more organic $C_1$-$C_6$ alkoxy-silanes of formula (II)

$$R_2\text{-Si}(OR_3)_3 \qquad (II)$$

where

- $R_2$ represents a $C_1$-$C_{12}$-alkyl group, and
- $R_3$ represents a $C_1$-$C_6$-alkyl group, and

(a3) water, and, based on the total weight of the composition

(b) one or more $C_1$-$C_6$ alcohols in a total amount of 0.001 to 10.0% by weight; and
(c) 0.001 to 10.0% by weight of water.

**2.** Composition according to claim 1, comprising

(a) a product obtained by mixing

(a1) (3-aminopropyl)triethoxysilane with
(a2) methyltrimethoxysilane and
(a3) water.

**3.** Composition according to claim 1, comprising

(a) a product obtained by mixing

(a1) (3-aminopropyl)triethoxysilane with
(a2) ethyltriethoxysilane and
(a3) water.

**4.** Composition according to claim 1, comprising

(a) a product obtained by mixing

(a1) (3-aminopropyl)triethoxysilane with
(a2) methyltriethoxysilane and
(a3) water.

**5.** Composition according to claim 1, comprising

(a) a product obtained by mixing

(a1) (3-aminopropyl)triethoxysilane with
(a2) propyltriethoxysilane and
(a3) water.

**6.** Composition according to claim 1, comprising

(a) a product obtained by mixing

(a1) (3-aminopropyl)triethoxysilane with
(a2) hexyltriethoxysilane and
(a3) water.

**7.** Composition according to claim 1, comprising

(a) a product obtained by mixing

(a1) (3-aminopropyl)triethoxysilane with
(a2) octyltriethoxysilane and
(a3) Water.

**8.** Composition according to any one of claims 1 to 7, **characterized in that** the organic $C_1$-$C_6$-alkoxy silanes (a1) and the organic $C_1$-$C_6$-alkoxy silanes (a2) are mixed with one another to give the product (a) in a molar ratio (a2)/(a1) of from 3.0 to 8.0, preferably from 3.5 to 7.5, more preferably from 4.0 to 7.0, still more preferably from 4.5 to 6.5 and very particularly preferably from 5.4 to 5.9.

**9.** Composition according to any one of claims 1 to 8, **characterized in that** the organic $C_1$-$C_6$-alkoxy silanes (a1) and water (a3) are mixed with each other to obtain the product (a) in a molar ratio (a3)/(a1) of from 4.0 to 9.5, preferably from 4.5 to 9.0, more preferably from 5.0 to 8.5, still more preferably from 5.5 to 8.0 and most preferably from 5.8 to 6.4.

**10.** Composition according to any one of claims 1 to 9, **characterized in that** the organic $C_1$-$C_6$-alkoxy silanes (a2) and (a3) water are mixed together to obtain the product (a) in a molar ratio (a3)/(a2) of from 0.1 to 3.5, preferably from 0.3 to 3.0, more preferably from 0.5 to 2.5, still more preferably from 0.7 to 2.0, and most preferably from 0.9 to 1.3.

**11.** Composition according to any one of claims 1 to 10, comprising - based on the total weight of the composition - one or more products (a) in a total amount of from 30.0 to 85.0% by weight, preferably from 35.0 to 80.0% by weight, more preferably from 40.0 to 75.0% by weight, still more preferably from 45.0 to 70.0% by weight, and most preferably from 50.0 to 65.0% by weight.

**12.** Composition according to any one of claims 1 to 11, comprising - based on the total weight of the composition -.
(b) one or more $C_1$-$C_6$ alcohols in a total amount of from 0.01 to 9.0% by weight, preferably from 0.1 to 8.0% by weight, more preferably from 0.5 to 7.0% by weight, and most preferably from 0.5 to 5.0% by weight.

**13.** Composition according to any one of claims 1 to 12, comprising - based on the total weight of the composition - (b1) from 0.01 to 9.0% by weight, preferably from 0.01 to 8.0% by weight, more preferably from 0.01 to 7.0% by weight and most preferably from 0.01 to 4.0% by weight of methanol.

**14.** Composition according to any one of claims 1 to 13, comprising - based on the total weight of the composition - (b2) from 0.01 to 9.0% by weight, preferably from 0.1 to 8.0% by weight, more preferably from 0.5 to 7.0% by weight and most preferably from 0.5 to 4.0% by weight of ethanol.

**15.** Composition according to any one of claims 1 to 14, comprising - based on the total weight of the composition -
(c) 0.01 to 9.0% by weight, preferably 0.1 to 7.0% by weight, more preferably 0.2 to 5.0% by weight and most preferably 0.5 to 3.0% by weight water.

**16.** Composition according to any one of claims 1 to 15, comprising
(d) one or more cosmetic ingredients selected from the group consisting of hexamethyldisiloxane, octamethyltrisiloxane, decamethyltetrasiloxane, hexamethylcyclotrisiloxane, octamethylcyclotetrasiloxane and decamethylcyclopentasiloxane.

**17.** Composition according to any one of claims 1 to 16, comprising - based on the total weight of the composition - (d) 10.0 to 50.0% by weight, preferably 15.0 to 45.0% by weight, more preferably 20.0 to 40.0% by weight, still more preferably 25.0 to 35.0% by weight, and most preferably 31.0 to 34.0% by weight of hexamethyldisiloxane.

**18.** Composition according to any one of claims 1 to 17, **characterized in that**, after dilution with distilled water in a weight ratio of 1:1, it has a pH of from 7.0 to 12.0, preferably from 7.5 to 11.5, more preferably from 8.5 to 11.0, and most preferably from 9.0 to 11.0.

**19.** Multi-component packaging unit (kit-of-parts) for dyeing keratinous material, in particular human hair, which are made up separately from each other.

- a first packaging unit containing a cosmetic preparation (A) and
- a second packaging unit containing a cosmetic preparation (B),

where

- the cosmetic preparation (A) is a composition according to any one of claims 1 to 18, and
- the cosmetic formulation (B) comprises at least one colorant compound selected from the group consisting of pigments and/or direct dyes.

**20.** Multi-component packaging unit (kit-of-parts) according to claim 19, comprising.

- a third packaging unit containing a cosmetic preparation (C), wherein the cosmetic preparation (C) contains at least one thickening polymer

**21.** Multi-component packaging unit (kit-of-parts) according to any one of claims 19 to 20, comprising

- a fourth packaging unit containing a cosmetic preparation (D), wherein the cosmetic preparation (D) contains at least one film-forming polymer

**Revendications**

**1.** Composition cosmétique pour le traitement des matières kératiniques, en particulier des fibres kératiniques, contenant

(a) un produit obtenu par mélange de

(a1) un ou plusieurs $C_1$-$C_6$-alcoxy-silanes organiques de formule (I)

$$H_2N\text{-}L\text{-}Si(OR_1)_3 \quad (I)$$

où

- L représente un groupe alkylène divalent en $C_1$-$C_{20}$ linéaire ou ramifié,
- $R_1$ représente un groupe alkyle en $C_1$-$C_6$,

avec
(a2) un ou plusieurs $C_1$-$C_6$-alcoxy-silanes organiques de formule (II)

$$R_2\text{-}Si(OR_3)_3 \quad (II)$$

où

- $R_2$ représente un groupe alkyle en $C_1$ -$C_{12}$ , et
- $R_3$ représente un groupe alkyle en $C_1$-$C_6$, et

(a3) de l'eau et, sur la base du poids total de la composition

(b) un ou plusieurs alcools $C_1$-$C_6$ en une quantité totale de 0,001 à 10,0 % en poids, et
(c) 0,001 à 10,0 % en poids d'eau.

**2.** Composition selon la revendication 1, contenant

(a) un produit obtenu par mélange de

(a1) (3-aminopropyl)triéthoxysilane avec
(a2) méthyltriméthoxysilane ; et
(a3) eau.

**3.** Composition selon la revendication 1, contenant

(a) un produit obtenu par mélange de

(a1) (3-aminopropyl)triéthoxysilane avec
(a2) éthyltriéthoxysilane; et
(a3) eau.

**4.** Composition selon la revendication 1, contenant

(a) un produit obtenu par mélange de

(a1) (3-aminopropyl)triéthoxysilane avec
(a2) méthyltriéthoxysilane; et
(a3) eau.

**5.** Composition selon la revendication 1, contenant

(a) un produit obtenu par mélange de

(a1) (3-aminopropyl)triéthoxysilane avec
(a2) propyltriéthoxysilane; et
(a3) eau.

**6.** Composition selon la revendication 1, contenant

(a) un produit obtenu par mélange de

(a1) (3-aminopropyl)triéthoxysilane avec
(a2) hexyltriéthoxysilane; et
(a3) eau.

**7.** Composition selon la revendication 1, contenant

(a) un produit obtenu par mélange de

(a1) (3-aminopropyl)triéthoxysilane avec
(a2) octyltriéthoxysilane; et
(a3) eau.

**8.** Composition selon l'une quelconque des revendications 1 à 7, **caractérisée en ce que** les organiques $C_1$-$C_6$-alcoxy-silanes (a1) et les $C_1$ -$C_6$ -alcoxy-silanes organiques (a2) sont mélangés entre eux dans un rapport molaire (a2)/(a1) de 3,0 à 8,0, de préférence de 3,5 à 7,5, plus préférablement de 4,0 à 7,0, encore plus préférablement de 4,5 à 6,5 et tout particulièrement de 5,4 à 5,9, pour obtenir le produit (a).

**9.** Composition selon l'une quelconque des revendications 1 à 8, **caractérisée en ce que** les organiques $C_1$-$C_6$-alcoxy-silanes (a1) et de l'eau (a3) pour obtenir le produit (a) dans un rapport molaire (a3)/(a1) de 4,0 à 9,5, de préférence de 4,5 à 9,0, plus préférablement de 5,0 à 8,5, encore plus préférablement de 5,5 à 8,0 et tout particulièrement de 5,8 à 6,4.

**10.** Composition selon l'une quelconque des revendications 1 à 9, **caractérisée en ce que** les $C_1$-$C_6$-alcoxy-silanes organiques (a2) et (a3) eau sont mélangés ensemble pour obtenir le produit (a) dans un rapport molaire (a3)/(a2) de 0,1 à 3,5, de préférence de 0,3 à 3,0, plus préférentiellement de 0,5 à 2,5, encore plus préférentiellement de 0,7 à 2,0 et tout particulièrement de 0,9 à 1,3.

**11.** Composition selon l'une quelconque des revendications 1 à 10, contenant - par rapport au poids total de la composition - un ou plusieurs produits (a) en une quantité totale de 30,0 à 85,0 % en poids, de préférence de 35,0 à 80,0 % en poids, plus préférablement de 40,0 à 75,0 % en poids, encore plus préférablement de 45,0 à 70,0 % en poids et tout particulièrement de 50,0 à 65,0 % en poids.

**12.** Composition selon l'une quelconque des revendications 1 à 11, contenant - par rapport au poids total de la composition -

(b) un ou plusieurs alcools $C_1$ -$C_6$ en une quantité totale de 0,01 à 9,0 % en poids, de préférence de 0,1 à 8,0 % en poids, plus préférablement de 0,5 à 7,0 % en poids et tout particulièrement de 0,5 à 5,0 % en poids.

**13.** Composition selon l'une quelconque des revendications 1 à 12, contenant - par rapport au poids total de la composition - (b1) de 0,01 à 9,0 % en poids, de préférence de 0,01 à 8,0 % en poids, plus préférentiellement de 0,01 à 7,0 % en poids et tout particulièrement de 0,01 à 4,0 % en poids de méthanol.

**14.** Composition selon l'une quelconque des revendications 1 à 13, contenant - par rapport au poids total de la composition - (b2) de 0,01 à 9,0 % en poids, de préférence de 0,1 à 8,0 % en poids, plus préférentiellement de 0,5 à 7,0 % en poids et tout particulièrement de 0,5 à 4,0 % en poids d'éthanol.

**15.** Composition selon l'une quelconque des revendications 1 à 14, contenant -par rapport au poids total de la composition (c) 0,01 à 9,0 % en poids, de préférence 0,1 à 7,0 % en poids, plus préférablement 0,2 à 5,0 % en poids et tout particulièrement 0,5 à 3,0 % en poids d'eau.

**16.** Composition selon l'une quelconque des revendications 1 à 15, contenant
(d) un ou plusieurs ingrédients cosmétiques choisis dans le groupe constitué par l'hexaméthyldisiloxane, l'octaméthyltrisiloxane, le décaméthyltétrasiloxane, l'hexaméthylcyclotrisiloxane, l'octaméthylcyclotétrasiloxane et le décaméthylcyclopentasiloxane.

**17.** Composition selon l'une quelconque des revendications 1 à 16, contenant - par rapport au poids total de la composition - (d) 10,0 à 50,0 % en poids, de préférence 15,0 à 45,0 % en poids, plus préférentiellement 20,0 à 40,0 % en poids, encore plus préférentiellement 25,0 à 35,0 % en poids et tout particulièrement 31,0 à 34,0 % en poids d'hexaméthyldisiloxane.

**18.** Composition selon l'une quelconque des revendications 1 à 17, **caractérisée en ce que**, après dilution avec de l'eau distillée dans un rapport pondéral de 1:1, elle possède un pH de 7,0 à 12,0, de préférence de 7,5 à 11,5, plus préférentiellement de 8,5 à 11,0 et tout particulièrement de 9,0 à 11,0.

**19.** Unité d'emballage à plusieurs composants (kit of parts) pour la coloration de matières kératiniques, en particulier de cheveux humains, qui sont confectionnés séparément les uns des autres

- une première unité d'emballage contenant une préparation cosmétique (A) et
- une deuxième unité d'emballage contenant une préparation cosmétique (B),

où

- la préparation cosmétique (A) est une composition selon l'une des revendications 1 à 18 et
- la préparation cosmétique (B) contient au moins un composé colorant du groupe des pigments et/ou des colorants à action directe.

**20.** Unité d'emballage à plusieurs composants (kit-of-parts) selon la revendication 19, comprenant

- une troisième unité d'emballage contenant une préparation cosmétique (C), la préparation cosmétique (C) contenant au moins un polymère épaississant.

**21.** Unité d'emballage à plusieurs composants (kit-of-parts) selon l'une quelconque des revendications 19 à 20, comprenant

- une quatrième unité d'emballage contenant une préparation cosmétique (D), la préparation cosmétique (D) contenant au moins un polymère filmogène.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2168633 B1
- WO 2013068979 A2
- WO 2012038880 A2